(19) Europäisches Patentamt
European Patent Office
Office européen des brevets

(11) EP 1 835 812 B1

(12) # EUROPÄISCHE PATENTSCHRIFT

(45) Veröffentlichungstag und Bekanntmachung des Hinweises auf die Patenterteilung:
**17.08.2011 Patentblatt 2011/33**

(21) Anmeldenummer: **05850500.9**

(22) Anmeldetag: **27.12.2005**

(51) Int Cl.:
***A61K 8/41*** *(2006.01)* ***A61Q 15/00*** *(2006.01)*
***A61K 8/36*** *(2006.01)* ***A61K 8/33*** *(2006.01)*

(86) Internationale Anmeldenummer:
**PCT/EP2005/057186**

(87) Internationale Veröffentlichungsnummer:
**WO 2006/069998 (06.07.2006 Gazette 2006/27)**

(54) **GLYCOPYRROLAT IN KOSMETISCHEN ZUBEREITUNGEN**

GLYCOPYRROLATE IN COSMETIC PREPARATIONS

LE COMPOSE GLYCOPYRROLATE DANS DES PREPARATIONS COSMETIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.12.2004 DE 102004063728**
**27.12.2004 DE 102004063726**
**23.06.2005 DE 102005029390**
**23.06.2005 DE 102005029388**
**23.06.2005 DE 102005029386**
**23.06.2005 DE 102005029387**
**23.06.2005 DE 102005029385**
**23.06.2005 DE 102005029389**

(43) Veröffentlichungstag der Anmeldung:
**26.09.2007 Patentblatt 2007/39**

(73) Patentinhaber: **Beiersdorf AG**
**20253 Hamburg (DE)**

(72) Erfinder:
- **CERV, Svenja-kathrin**
**22761 Hamburg (DE)**
- **THOMAS, Raschke**
**25421 Pinneberg (DE)**
- **BIEL, Stefan**
**21075 Hamburg (DE)**
- **TERSTEGEN, Lara**
**20253 Hamburg (DE)**
- **WÖHRMANN, Michael**
**22525 Hamburg (DE)**
- **MAX, Heiner**
**22529 Hamburg (DE)**
- **UWE, Schönrock**
**23866 Nahe (DE)**
- **NÜBEL, Thomas**
**22525 Hamburg (DE)**
- **UNTIEDT, Sven**
**20257 Hamburg (DE)**
- **ZILZ, Werner**
**25469 Halsenbek (DE)**
- **BIERGIESSER, Helga**
**21465 Reinbek (DE)**
- **KRUSE, Inge**
**20146 Hamburg (DE)**
- **TRAUPE, Bernd**
**24568 Kaltenkirchen (DE)**
- **MEIER-ZIMMERER, Cornelia**
**20529 Hamburg (DE)**
- **FÖLSTER, Heike**
**20257 Hamburg (DE)**
- **MIERTSCH, Heike**
**22529 Hamburg (DE)**
- **CIERPISZ, Yvonne**
**20251 Hamburg (DE)**

(56) Entgegenhaltungen:
**WO-A-86/02272**
**WO-A-03/026585**
**WO-A-2004/093792**
**DE-A1- 19 516 705**
**GB-A- 1 080 960**
**US-A1- 2001 036 450**
**US-A1- 2003 211 134**
**US-B1- 6 723 311**

Bemerkungen:
Die Akte enthält technische Angaben, die nach dem Eingang der Anmeldung eingereicht wurden und die nicht in dieser Patentschrift enthalten sind.

EP 1 835 812 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Der Mensch besitzt zwei unterschiedliche Formen von Schweißdrüsen. Die ekkrinen Schweißdrüsen sondern hauptsächlich Salz und Wasser ab und tragen üblicherweise nicht zur Geruchsbildung bei. Für den Geruch sind die apokrinen Schweißdrüsen verantwortlich, die Fettsäuren, Cholesterine und andere Verbindungen ausscheiden. Diese Substanzen werden von Bakterien auf der Haut zersetzt, wobei die Abbauprodukte den für Schweiß typischen Geruch erzeugen.

Um den Schweißgeruch über einen längeren Zeitraum zu unterdrücken, ist der Einsatz kosmetischer Zubereitungen unerlässlich. Den üblichen kosmetischen Desodorantien liegen unterschiedliche Wirkprinzipien zugrunde, die auch kombiniert werden können: Zum einen werden Deowirkstoffe eingesetzt, die das Wachstum der den Schweißgeruch verursachenden Bakterien unterdrücken. Zu diesen keimhemmenden (bakteriostatischen) Mitteln zählen beispielsweise Triclosan, Chlorhexidin oder die natürlich vorkommenden Verbindungen wie Farnesol und Phenoxyethanol.

Zum anderen werden Antitranspirantien eingesetzt, welche die Schweißabsonderung durch Blockierung der Schweißdrüsenausgänge behindern. In den weitaus meisten Antitranspirantien kann durch Adstringentien - vorwiegend Aluminiumsalze wie Aluminiumhydroxychlorid (Aluchlorhydrat) oder Aluminium/Zirkoniumsalze - die Bildung des Schweißes reduziert werden. Auch die Kombination von Adstringentien mit antimikrobiell wirksamen Stoffen in ein und derselben Zusammensetzung ist gebräuchlich. Ferner werden Parfümstoffe zur Überdeckung des Schweißgeruches eingesetzt.

Nachteilig bei der Verwendung von Aluminiumchlorhydrat ist beispielsweise, dass Rückstände die Kleidung in unschöner Weise verfärben können und der niedrige pH-Wert (sauer) der kosmetischen Zubereitung das biologische Gleichgewicht der Haut unvorteilhaft beeinflusst.

Bekannt und gebräuchlich sind neben den flüssigen Desodorantien wie Zerstäuber und Roll-on auch feste Zubereitungen, beispielsweise Deo-Stifte ("Sticks"), Puder, Pudersprays, Intimreinigungsmittel usw.

An ein zufriedenstellendes Deo/AT-Mittel, bevorzugt nur Desodorantien, werden folgende Voraussetzungen geknüpft, deren Verwirklichung die Aufgabe der vorliegenden Erfindung darstellt: 1) Schonung der natürlichen Biologie der Haut 2) Duftneutralität 3) Wirksamkeit nur in Bezug auf Desodorierung, d.h. nur Vermeidung und/oder Beseitigung von Körpergeruch 4) Vermeidung der Bildung von resistenten Bakterienstämmen 5) Vermeidung der Akkumulation der Wirkstoffe auf der Haut 6) Unschädlichkeit bei Überdosierung oder sonstiger nicht bestimmungsgemäßer Anwendung 7) Gute kosmetische Anwendung 8) Leichte Handhabung (z.B. als Flüssigkeit) und universelle Verwendbarkeit in verschiedensten kosmetischen und externen Zubereitungen 9) Ausgezeichnete Haut- und Schleimhautverträglichkeit 10) Einsatz umweltfreundlicher Stoffe.

Glycopyrroniumbromid (Internationaler Freiname für (±)-(R*)-3-[(S*)-(Cyclopentyl-hydroxyphenylacetoxyl-1,1-dimethylpyrrolidiniumbromid) ist ein seit 1960 bekanntes Anticholinergikum und Spasmolytikum. Es wird, vor allem im englischen Sprachgebrauch als "Glycopyrrolat" bezeichnet und zeichnet sich durch folgende chemische Struktur aus:

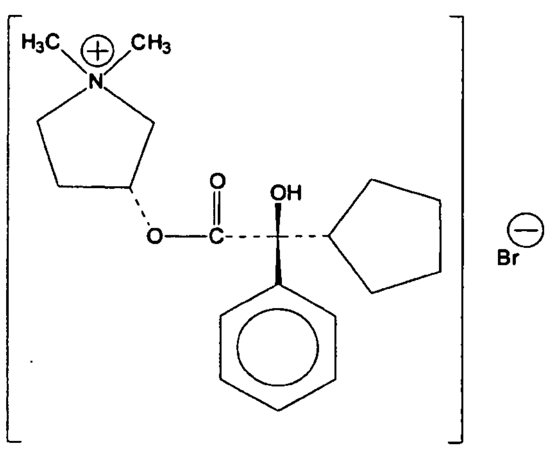

Glycopyrrolat zeigt keine oder nur geringe antimikrobielle Wirksamkeit.

Im Stand der Technik wird in erster Linie auf die Verwendung Glycopyrrolat-haltiger Zusammensetzungen zur Behandlung von pathologischem Schwitzen wie primärer oder sekundärer Hyperhidrosis, gustatorischem Schwitzen (Frey-Syndrom) abgestellt.

In Anaesthesia, 1983, Volume 38, Seiten 1195-1204, wird die Verwendung von Glycopyrrolat in der Anästhesie beschrieben. Es wird ein Überblick über die pharmakologischen Aspekte von Glycopyrrolat gegeben, wobei die anticholinergen Eigenschaften und die Eignung, die Schweißdrüsenaktivität zu vermindern, erwähnt werden.

In L.V.Allen, "Topical Agent Stops Facial Sweating", Pharmacist, July 1998, wird eine Glycopyrrolat und weitere pharmazeutische/kosmetische Hilfsstoffe enthaltende Formulierung zur Behandlung von gustatorischem Schwitzen und zur Behandlung des Frey-Syndroms offenbart.

In C.L. Hays et al., "The Frey Syndrome: A Simple, Effective Treatment", Otolaryngol Head Neck Surg. 1982, 90, 419-425, wird eine klinische Vergleichsstudie zur topischen Behandlung von gustatorischem Schwitzen (Frey-Syndrom) mit Scopolamin und Glycopyrrolat beschrieben. Darin befasst man sich mit dem pathologischen Schwitzen. Bezogen auf den Wirkungsmechanismus von Glycopyrrolat wird offenbart, dass Glycopyrrolat den zur Primärschweißbildung führenden Nervenreiz der Schweißzellen der Haut blockiert, und dabei weniger Nebenwirkungen als Scopolamin zeigt, Da es als quarternäre Ammoniumverbindung nicht die Blut-Hirn-Schranke passiert und biologische Membranen langsamer durchdringt.

Die WO03/026585 beschreibt die Verwendung von Glycopyrroniumbromid zur Hemmung der ekkrinen Transpiration beim Menschen - wiewohl die Wirkung von Glycopyrroniumbromid und dessen Verwendung gegen Hyperhidrosis schon deutlich länger bekannt ist.

Die GB 1080960 A1 beschreibt Antitzranspirantzubereitungen umfassend Glycopyrroniumbromid.

WO-A-01/08681 offenbart die Behandlung einer Reihe von pathologischen Zuständen mit Zusammensetzungen auf Basis von Glycopyrrolat, einem speziellen anticholinergen Amin. Die Zustände sind u.a. gustatorisches Schwitzen ("Geschmacksschwitzen") sowie das "Frey-Syndrom", worunter man verstärkte Schweißbildung im Ohr-Schläfen-Bereich versteht, die durch örtliche Reizung sowie durch bestimmte Speisen ausgelöst sein kann. Daneben wird Hyperhidrose (vermehrte Schweißabsonderung) erwähnt. Es werden Formulierungen offenbart, die für die topische Verabreichung auf der Haut geeignet sind, wie Salben, Cremes, Gele und Pasten, und die einen pharmazeutisch verträglichen Träger.

US 6 433 033 B1 offenbart Zusammensetzungen, die 0,25 bis 6 Gew.%, insbesondere 0,5 bis 4 Gew.% Glycopyrrolat zusammen mit weiteren pharmazeutisch akzeptablen Bestandteilen enthält. Diese Zusammensetzung wird zur Behandlung von Hyperhidrose verwendet und dabei topisch verabreicht.

WO-A-03/011340 offenbart eine Arzneiformulierung enthaltend Glycopyrrolat bzw. Salze oder Derivate davon in einer Menge zwischen 0,05 und 20 Gew.% und auch ein Vehikel wie ein Gel und/oder kolloidhaltiges Trägersystem. Die Glycopyrrolat-Formulierungen werden zur Behandlung der Hyperhidrose topisch verabreicht.

In der DE 19516705 A1 wird die antibakterielle Wirksamkeit von dialkyl-substituierten Essigsäuren beschrieben.

Aufgabe der vorliegenden Erfindung ist es eine kosmetische Zubereitung bereit zu stellen, die

- ➢ eine gesteigerte antitranspirierende Wirkung zeigt,
- ➢ eine gesteigerte desodorierende Wirkung zeigt
- ➢ hautpflegende Eigenschaft aufweist und sich durch bessere Pflegewirkung auszeichnet,
- ➢ eine sensorisch unerwünschte Klebrigkeit der Zubereitung vermeiden hilft,
- ➢ eine ungewünschte Eintrübung der Zubereitung vermeiden hilft,
- ➢ durch Hautneutralität das biologische Gleichgewicht der Haut weniger belastet,
- ➢ besser als Vehikel für kosmetische und medizinisch-dermatologische Wirkstoffe dient,
- ➢ sich durch eine bessere physikochemische Stabilität der Formel auszeichnet,
- ➢ sich durch bessere Bioverträglichkeit auszeichnet und/oder
- ➢ bessere sensorische Eigenschaften, wie beispielsweise die Verteilbarkeit oder das Einzugsvermögen in die Haut, besitzt,

als die Wirkstoffe, Wirkstoffkombinationen und Zubereitungen des Standes der Technik.

Gelöst wird dieses Bündel an Aufgaben durch eine kosmetische Zubereitung entsprechend den Hauptansprüchen. Gegenstand der Unteransprüche sind vorteilhafte Ausführungsformen der erfindungsgemäßen Zubereitungen sowie deren Verwendung.

Es war überraschend und für den Fachmann nicht vorauszusehen, dass eine Wirkstoffkombination und eine kosmetische Zubereitung diese umfassend, umfassend Glycopyrroniumbromid in Kombination mit einer oder mehrerer dialkyl-substituierter Essigsäuren, die Aufgaben lösen.

Bevorzugt werden die nachfolgenden Gewichtsverhältnisse eingestellt, wobei (A : B : C) wie a : b : c gewählt wird, wobei a, b und c unabhängig voneinander positive rationale Zahlen von 1 bis 200, bevorzugt von 1 bis 50 darstellen,

A stellt dabei die der Konzentration von Glycopyrroniumbromid in Gewichtseinheiten (z.B. Gew.-%) dar,
B stellt dabei die Konzentration eines Aktivstoffes in denselben Gewichtseinheiten dar,
C stellt dabei die Konzentration eines zweiten Aktivstoffes dar, gewählt aus der Gruppe
a.)Polyethylenglykol(2)stearylether und Polyethylenglykol(21)stearylether,
b.)grenzflächenaktiver Substanzen A, gewählt aus der Gruppe der Glucosederivate, welche
sich durch die Strukturformel

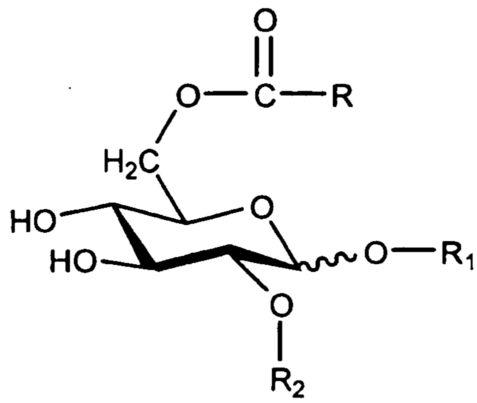

auszeichnen, wobei R einen verzweigten oder unverzweigten Alkylrest mit 1 bis 24 Kohlenstoffatomen darstellt, wobei R1 entweder ein Wasserstoffatom oder einen verzweigten oder unverzweigten Alkylrest mit 1 bis 24 Kohlenstoffatomen darstellt und wobei R2 entweder ein Wasserstoffatom oder einen verzweigten oder unverzweigten Acylrest mit 1 bis 24 Kohlenstoffatomen darstellt,
wobei zusätzlich eine oder mehrere grenzflächenaktive Substanzen B enthalten sein können, gewählt aus der Gruppe der Substanzen der allgemeinen Strukturformel

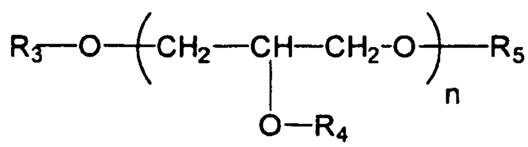

,

wobei R3, R4 und R5 voneinander unabhängig gewählt werden aus der Gruppe, welche umfasst: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt,
c.) eines oder mehrerer partiell neutralisierter Ester von Monoglyceriden und/oder Diglyceriden gesättigter Fettsäuren mit Zitronensäure,
d.)eines oder mehrerer Polyole aus der Gruppe umfassend Ethylenglykol, Glycerin, Octoxyglycerin (2-Ethylhexylglycerinether) und $C_4$-$C_{12}$ - Alkandiole, bevorzugt 1,2-Decandiol
e.) eines oder mehrerer Hydrokolloide, gewählt aus der Gruppe der organisch natürlichen oder modifiziert natürlichen, organisch vollsynthetischen oder anorganisch wasserlöslichen Polymere,
f.) eines oder mehrerer Mono-, Oligo- und/oder Polysaccharide,
g.)der Silikate in Kombination mit Ölen,
h.)der Öle, die gewählt werden aus aus der Gruppe der Alkylbenzoate, Esteröle, Dialkylcarbonate und -ether, lineare und/oder verzweigtkettige, aliphatische Kohlenwasserstoffe und/oder kurzkettige Kohlenwasserstoffester, insbesondere Isopropylester,
i.) der Parfumstoffe gewählt aus der Gruppe der Aldehyde, Ester, Jonone, Methyljonone, Damascone. Salicylate, Acetale und/oder Holzkörper, insbesondere Iso E Super,
j.) des Phenoxyethanols und/oder
k.) antimikrobiell wirksamer silberhaltiger Gläser, bevorzugt
in Form einer
m.) einer W/Si - Emulsion,
n.) eines O/W - Gels oder
o.) eines Seifengelstiftes.

Erfindungsgemäß wird im Folgenden als Glycopyrrolat oder Glycopyrroniumbromid (Internationaler Freiname für (±)-(R*)-3-[(S*)-(Cyclopenty)hydroxyphenylacetoxy]-1,1-dimethylpyrrolidiniumbromid) synonym bezeichnet.
Bevorzugt ist Glycopyrroniumbromid gekennzeichnet durch folgende chemische Struktur:

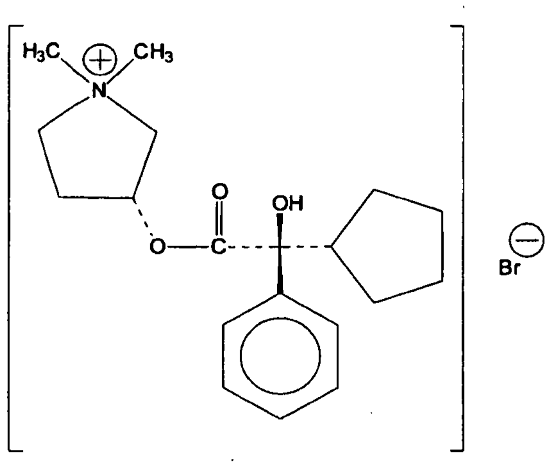

Glycopyrrolat hat keine oder nur geringe antimikrobielle Wirksamkeit. In Kombination mit den erfindungsgemäß ermittelten Aktivstoffen bzw. Formen a.) bis k.) zeigt sich aber überraschenderweise, dass sich die antimikrobielle Wirksamkeit und damit auch die Antitranspirant (AT)-wirksamkeit und insbesondere die desodorierende Wirksamkeit überdurchschnittlich erhöht.

Glycopyrrolat dient somit auch als Enhancer für antimikrobielle Wirkstoffe. Überraschend und für den Fachmann nicht zu erwarten konnte gezeigt werden, dass Glycopyrrolat allein in den verwendeten Formulierungen keine antibakterielle Wirkung zeigt, in Kombination mit anderen Wirkstoffen aber, wie insbesondere antimikrobiell wirkende Aluminiumsalze oder Silbersalze, aber zu einer signifikant besseren antimikrobiellen Wirkung führt als die Wirkstoffe allein ohne Glycopyrrolat.

Der Zusatz an Deo/AT-wirkern, wie antimikrobiell wirkenden Aluminiumsalze, wie z.B. Aluminumchlorohydrat (ACH), ist somit zwar bevorzugt aber nicht zwingend erforderlich.

Dieser Effekt beruht darauf, dass Glycopyrrolat sich ähnlich wie ein Surfactant in die Membran der Bakterien einlagert. Diese Einlagerung allein reicht zwar nicht aus, die Bakterien abzutöten, macht diese aber anfälliger für den Angriff durch antibakterielle Substanzen (Deo/AT-wirker), die durch die gestörte Membranintegrität diese leichter passieren oder sogar zerstören können.

Ferner hat es sich als vorteilhaft erwiesen, den Quotienten ( B + C)/A wobei A, B, und C die zuvor beschriebenen Eigenschaften aufweisen, aus dem Bereich zwischen 0,5 und 200, bevorzugt aus dem Bereich zwischen 1 und 50 zu wählen.

Bevorzugt liegen die erfindungsgemäßen Wirkstoffkombinationen in O/W-Emulsionen vor:

Der Zusatz von oberflächenaktiven Glucosederivaten und Oligoglycerinetherderivaten steigert die antitranspirierende Wirkung von Glycopyrroniumbromid in überraschender und nicht vorhersehbarer Weise.

Besonders vorteilhaft wird oder werden die oberflächenaktiven Glucosederivate A aus der Gruppe Methylglucosemonostearat (Formel wie nachstehend)

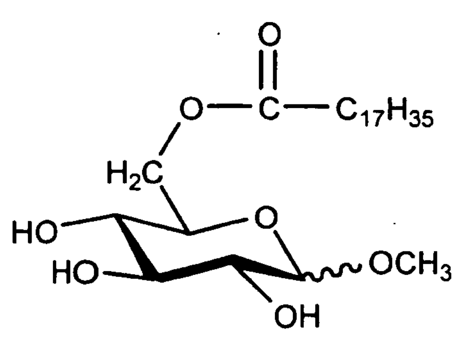

(A1)

oder Methylglucosedistearat (Formel wie nachstehend)

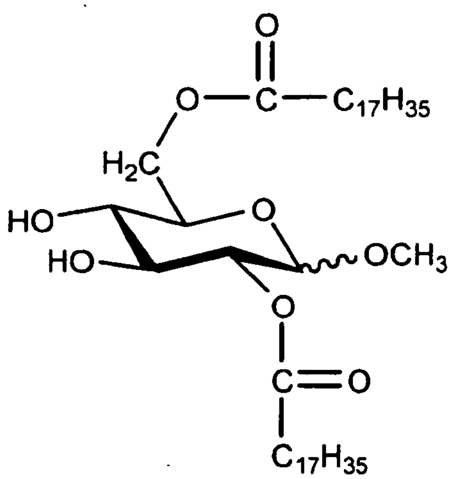

(A2),

und beliebige Gemische daraus, beispielsweise ungefähr äquimolare Mischungen daraus gewählt, welche auch als Methylglucosesesquistearat bezeichnet werden. Solches Methylglucosesesquistearat ist im Handel beispielsweise unter der Warenbezeichnung Tego® Care PS von der Gesellschaft Th.Goldschmidt KG erhältlich.

Besonders vorteilhaft wird oder werden die grenzflächenaktiven Substanzen B aus der Gruppe der Verbindungen gewählt, bei welcher n den Wert 3 annimmt und R3, R4 und R5 unabhängig voneinander gewählt werden aus der Gruppe, welche umfasst: H, verzweigte bzw. unverzweigte, gesättigte bzw. ungesättigte Fettsäurereste mit 14 bis 20 Kohlenstoffatomen, insbesondere die Triglyceryldicarboxylate mit der nachfolgend aufgeführten generellen Struktur:

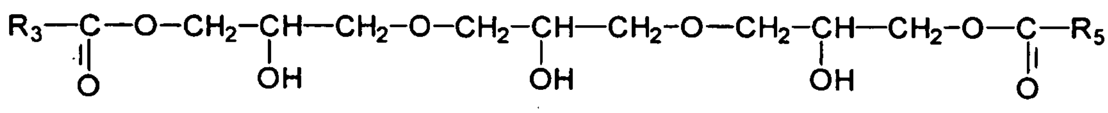

(B1),

Als erfindungsgemäß bevorzugte Emulgatorkombination hat sich ein ungefähr äquimolekulares Gemisch aus den Verbindungen A2 und B1, wobei in B1 die Reste R3 und R5 vorzugsweise beide einen Stearatrest bezeichnen, herausgestellt. Solche Emulgatorkombinationen sind als "Polyglyceryl(3)-Methylglucosedistearat" (PGMS) unter der Warenbezeichnung Tego Care® 450 von der Gesellschaft Th. Goldschmidt KG erhältlich.

Entsprechend der erfindungsgemäßen Verwendung sind die Desodorantien besonders vorteilhaft dadurch gekennzeichnet, dass die grenzflächenaktiven Substanzen A, gewählt aus der Gruppe der Glucosederivate, in Konzentrationen von 0,01 - 10,00 Gew.%, bevorzugt 0,05 - 5,00 Gew.%, besonders bevorzugt 0,1 - 3,00 Gew.%, jeweils bezogen auf das Gesamtgewicht der Zusammensetzung, vorliegen.

Entsprechend der erfindungsgemäßen Verwendung sind die Desodorantien besonders vorteilhaft dadurch gekennzeichnet, dass die grenzflächenaktiven Substanzen B, in Konzentrationen von 0,01 - 10,00 Gew.%, bevorzugt 0,05 - 5,00 Gew.%, besonders bevorzugt 0,1 - 3,00 Gew. %, jeweils bezogen auf das Gesamtgewicht der Zusammensetzung, vorliegt oder vorliegen.

Ein besonders vorteilhafter Zitronensäureester ist das Glycerylstearatcitrat. Solche Zitronensäureester sind beispielsweise erhältlich unter der Produktbezeichnung "IMWITOR® 370" der Firma SASOL.

Es ist erfindungsgemäß vorteilhaft, das molare Verhältnis von Glycopyrroniumbromid zu einem oder mehreren partiell neutralisierten Estern von Monoglyceriden und/oder Diglyceriden gesättigter Fettsäuren mit Zitronensäure aus dem Bereich von 100 : 1 bis 1 : 100 , bevorzugt 50 : 1 bis 1 : 50, insbesondere bevorzugt 20 : 1 bis 1 : 20 zu wählen.

Die erfindungsgemäßen Zubereitungen zeichnen sich durch eine hohe Wirksamkeit bei gleichzeitiger sehr guter Hautverträglichkeit aus. Außerdem haben sie ausgezeichnete sensorische Eigenschaften wie ein geringes Klebrigkeitsgefühl auf der Haut.

Erfindungsgemäß bevorzugte $C_4$-$C_{12}$ - Alkandiole sind die entsprechenden 1, 2-Diole mit unverzweigten Alkylketten, besonders bevorzugt ist 1,2-Decandiol.

Wie in Abbildung 2 dargestellt, zeigt die Kombination von 0,3 Gew.% GLYCOPYRROLAT und 0,15 Gew.% 1,2-Decandiol eine synergistische Erhöhung der Reduktion der Zellzahlen (C.xerosis) gegenüber den Zubereitungen

umfassend jeweils nur GLYCOPYRROLAT bzw. 1,2-Decandiol. Dies belegt wiederum eindrucksvoll den synergistischen Effekt der Kombinationen von GLYCOPYRROLAT mit den erfindungsgemäßen Aktivststoffen hinsichtlich Deo- und AT-wirkung.

Vorteilhafte Ausführungsformen der vorliegenden Erfindung sind allerdings auch 2-Methylpropandiol, 1,5-Pentandiol, 2,4-Pentandiol, 1,6-Hexandiol, 2,5-Hexandiol, 1,8-Octandiol, 1,10-Decandiol, 1,12-Dodecandiol.

Es ist erfindungsgemäß vorteilhaft, das molare Verhältnis von Glycopyrroniumbromid zu Polyol aus dem Bereich von 100 : 1 bis 1 : 100 , bevorzugt 50 : 1 bis 1 : 50, insbesondere bevorzugt 20 : 1 bis 1 : 20 zu wählen.

Entsprechend der erfindungsgemäßen Verwendung sind die Desodorantien besonders vorteilhaft dadurch gekennzeichnet, dass die Polyole in Konzentrationen von 0,01 - 20,00 Gew.%, bevorzugt 0,1 - 10,00 Gew.% jeweils bezogen auf das Gesamtgewicht der Zusammensetzung, vorliegt oder vorliegen.

Bekannt ist, dass bestimmte dialkyl-substituierte Essigsäuren aufgrund ihrer bakteriziden Wirkung auf eine Anzahl von Mikroorganismen als Konservierungsmittel eingesetzt werden. So ist aus der DE-OS 19516705 die Verwendung dialkyl-substituierter Essigsäuren als antibakterielle, antimycotische oder antivirale Wirkstoffe bekannt.

Es war nach all diesem aber dennoch überraschend und nicht vorhersehbar, dass Wirkstoffkombinationen aus Glycopyrroniumbromid und einer oder mehreren dialkyl-substituierter Essigsäuren, bzw. kosmetische Zubereitungen, solche Wirkstoffkombinationen enthaltend, sowie die Verwendung solcher Wirkstoffkombinationen als wirksames Prinzip kosmetischer Desodorantien und Antitranspirantien, die Nachteile des Standes der Technik beseitigen.

Erfindungsgemäß ist es daher möglich, zu desodorierend und/oder antitranspirant wirksamen Zubereitungen mit stärkerer Leistung zu gelangen, als es die Einzelsubstanzen erwarten ließen.

Vorteilhaft im Sinne der vorliegenden Erfindung sind dialkyl-substituierte Essigsäuren der Formel

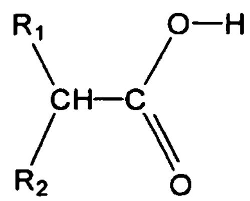

,

wobei $R_1$ einen verzweigten oder unverzweigten Alkylrest mit 1 - 12 Kohlenstoffatomen und $R_2$ einen verzweigten oder unverzweigten Alkylrest mit 1 - 24 Kohlenstoffatomen darstellt.

Vorteilhaft werden die Alkylreste so gewählt dass $R_1$ = Methyl, Ethyl, Propyl, Butyl, Pentyl oder Hexyl darstellt.

Weiter vorteilhaft werden die Alkylreste so gewählt, dass $R_2$= Octyl, Nonyl, Decyl, Undecyl, Dodecyl darstellt.

Insbesondere vorteilhaft ist, die erfindungsgemäßen Essigsäurederivate aus der Gruppe 2-Butyloctansäure, 2-Butyldecansäure, 2-Hexyloctansäure, 2-Hexyldecansäure zu wählen.

Ganz besonders bevorzugt ist die 2-Butyloctansäure.

Es ist erfindungsgemäß vorteilhaft, das molare Verhältnis von Glycopyrroniumbromid zu dialkyl-substituierten Essigsäuren aus dem Bereich von 100 : 1 bis 1 : 100 , bevorzugt 50 : 1 bis 1 : 50, insbesondere bevorzugt 20 : 1 bis 1 : 20 zu wählen.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

$-NH_2$ $-COOH$ $-COO^-$ $M^+$

$-NH-R$ $-SO_3^-$ $M^+$

$-OH$ $M^{2+}$

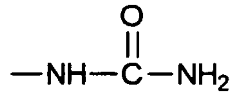

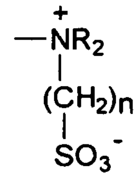

$-SH$ $-NH_3^+$ $-NR_2H$ $X^-$

$-O-$ $X^-$

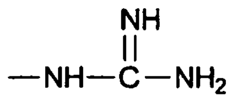

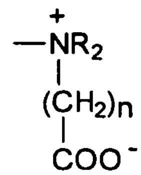

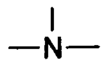

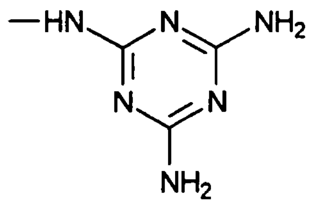

$X^-$

$-PR_3$ $X^-$

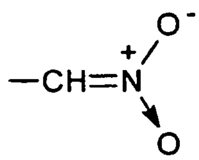

Die Gruppe der kosmetisch und dermatologisch relevanten Hydrokolloide lässt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane
- anorganische Verbindungen, wie z. B. Polykieselsäuren, Tonmineralien wie Montmorillonite, Zeolithe, Kieselsäuren.

Mikrokristalline Cellulose ist ein vorteilhaftes Hydrokolloid im Sinne der vorliegenden Erfindung. Sie ist beispielsweise von der "FMC Corporation Food and Pharmaceutical Products" unter der Handelsbezeichnung Avicel® erhältlich. Ein besonders vorteilhaftes Produkt im Sinne der vorliegenden Erfindung ist der Typ Avicel® RC-591, bei dem es sich um modifizierte mikrokristalline Cellulose handelt, die sich zu 89% aus mikrokristalliner Cellulose und zu 11 % aus Natrium Carboxymethyl Cellulose zusammensetzt. Weitere Handelsprodukte dieser Rohstoffklasse sind Avicel® RC/CL, Avicel® CE-15 und Avicel® 500.

Weitere erfindungsgemäß vorteilhafte Hydrokolloide sind beispielsweise Methylcellulosen, als welche die Methylether der Cellulose bezeichnet werden. Sie zeichnen sich durch die folgende Strukturformel aus

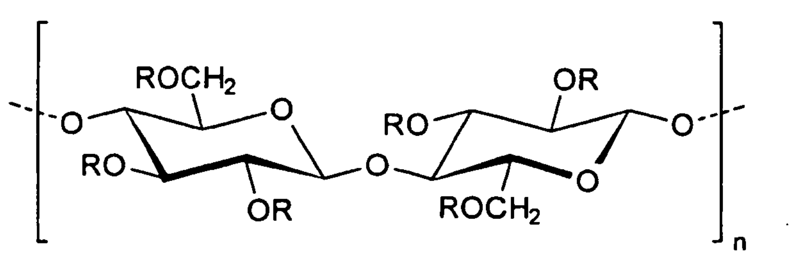

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel® E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder $CH_2$-CO0Na darstellen kann. Besonders bevorzugt sind hydrophob-modifizierte Hydroxyethylcellulosen, wie sie unter der Handelsbezeichnung Natrosol® Plus 330 CS bei Aqualon erhältlich sind. Weitere erfindungsgemäße Cellulosederivate sind die Ethylhydroxyethylcellulose (Elfacos CD 481 der Firma Akzo Nobel).

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von *Xanthomonas campestris* und einigen anderen Spezies unter aeroben Bedingungen mit einem Molekulargewicht von $2 \times 10^6$ bis $24 \times 10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans.

Vorteilhafter Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen *(Chondrus crispus* und *Gigartina stellata).*

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000 - 800 000 und einem Sulfat-Gehalt von ca. 25 %. Carrageen ist in warmem Wasser sehr leicht löslich und bildet beim Abkühlen ein thixotropes Gel, selbst wenn der Wassergehalt 95 - 98 % beträgt. Die Festigkeit des Gels wird durch die Doppelhelix-Struktur des Carrageens bewirkt. Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet: α, β, γ, μ, ν, ξ, π, ω, χ. Auch die Art vorhandener Kationen ($K^+$, $NH_4^+$, $Na^+$, $Mg^{2+}$, $Ca^{2+}$) beeinflusst die Löslichkeit der Carrageene.

Die Verwendung von Chitosan in kosmetischen Zubereitungen ist per se bekannt. Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, Da sie vom Verbraucher nicht akzeptiert bzw. negativ beurteilt werden. Chitosan wird bekanntermaßen beispielsweise in der Haarpflege eingesetzt. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Chitosan ist gekennzeichnet durch folgende Strukturformel:

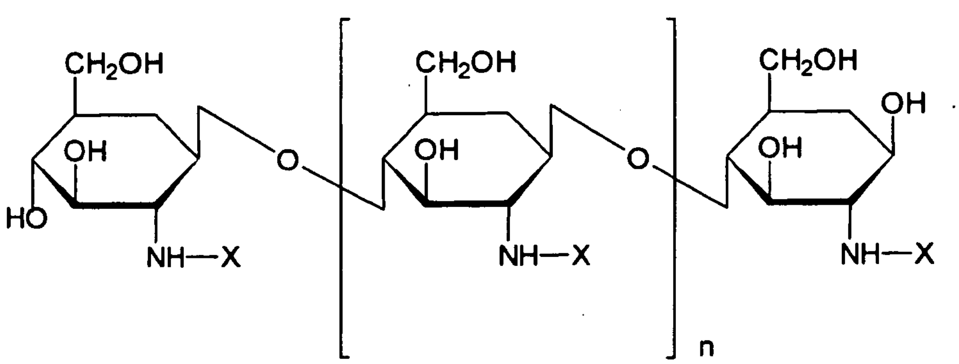

dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel

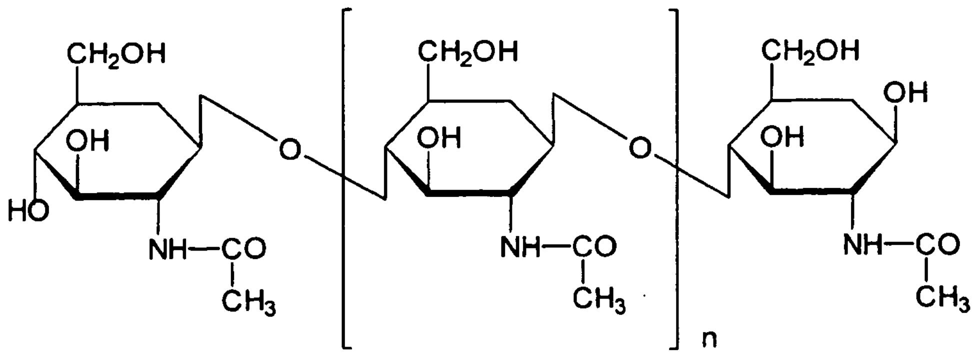

gekennzeichnet ist. Chitin ist wesentlicher Bestandteil des Ektoskeletts [το χιτων = griech.: der Panzerrock] der Gliederfüßler (z.B. Insekten, Krebse, Spinnen) und wird auch in Stützgeweben anderer Organismen (z.B. Weichtiere, Algen, Pilze) gefunden.

Im Bereich von etwa pH <6 ist Chitosan positiv geladen und dort auch in wässrigen Systemen löslich. Es ist nicht kompatibel mit anionischen Rohstoffen, daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657.

Erfindungsgemäß bevorzugt sind Chitosane mit einem Deacetylierungsgrad > 25 %, insbesondere > 55 - 99 % [bestimmt mittels $^{1}$H-NMR]).

Insbesondere sind Chitosan oder Chitosanderivate bevorzugt mit

A einem Deacetylierungsgrad von 75% - 98%, bevorzugt 78 - 86%, insbesondere 75 - 85%, 82 - 85% oder 82 - 84%, sowie 80 - 98%, bevorzugt 92 - 98%.

B einer Viskosität - zu 10 mPas, insbesondere von 4 - 10 mPas, bevorzugt 5 - 8 mPas,

C einem Gewichtsmittel der Molekulargewichtsverteilung von weniger als 300.000 Da, bevorzugt weniger als 160.000 Da oder weniger als 150.000 Da, insbesondere bevorzugt weniger als 100.000 Da, und bevorzugt zwischen 20.000 - 300.000 Da, insbesondere 50.000 - 160.000 Da, insbesondere 50.000 - 120.000 Da und

D einem Zahlenmittel der Molekulargewichtsverteilung kleiner 40.000 Da, bevorzugt kleiner 35.000 Da, insbesondere kleiner 27.000 Da, besonders bevorzugt 10.000 - 35.000 Da, insbesondere 10.000 - 27.000 Da.

Es ist auch von Vorteil, Chitosane mit Molekulargewichten zwischen 10.000 und 1.000.000 zu wählen, insbesondere solches mit Molekulargewichten zwischen 100.000 und 1.000.000 (bestimmt mittels Gelpermeationschromatographie).

Die erfindungsgemäß wesentlichen Parameter des Chitosans werden wie folgt bestimmt.

Die Viskosität einer 1 % Lösung wird nach Brookfield in 1 %iger Essigsäure bei 25˚C gemessen.

Das Molekulargewicht wird entsprechend der GLYCOPYRROLATC-Analyse in einer 0,1 molaren NaCl/0,1 Vol.%-Trifluoroessigsäure Lösung bei 23˚C mit einer Säule PSS-Novema (10 µm, linear, ID 8,0 mmx300 mm), einem Fluss von 1,0 ml/min., einer Probenkonzentration von 2,0 g/l und einem Injektionsvolumen von 20 µl bestimmt.

Der Deacetylierungsgrad lässt sich über die Direkttitrationsmethode bestimmen.

Die besonders effizient wirkenden Chitosane haben ein Molekulargewicht zwischen 10.000 bis 300.000 Da, insbesondere zwischen 50.000 -160.000 Da, ein Deacetylierungsgrad von 75 - 98%, eine Viskosität von maximal 10 mPas, ein Zahlenmittel der Molekulargewichtsverteilung kleiner 40.000 Da und setzen sich aus mindestens 50 Monomeren zusammen.

Als besonders bevorzugt sind Chitosane mit einem Molekulargewicht kleiner 160.000 Da, einem Deacetylierungsgrad von 78 - 86%, einer Viskosität von maximal 10 mPas und einem Zahlenmittel der Molekulargewichtsverteilung kleiner 35.000 Da.

Vorteilhaft zeigten sich insbesondere Chitosane mit einem Molekulargewicht kleiner 100.000 Da, einem Deacetylierungsgrad von 78 - 84%, einer Viskosität von maximal 10 mPas und einem Zahlenmittel der Molekulargewichtsverteilung kleiner 27.000 Da.

Erfindungsgemäß lassen sich für den speziellen Anwendungszweck erfindungsgemäße Chitosane mit den folgenden Kombinationen der bevorzugten Parameterbereichen A, B, C und D individuell auswählen, herstellen und anwenden.

Tabelle: Chitosanparameterbereiche

| A | B | C | D |
|---|---|---|---|
| Deacetylierungsgrad | Viskosität | Gewichtsmittel Molekulargewichtsverteilung | Zahlenmittel Molekulargewichtsverteilung |
| [%] | [mPas] | [Da] | [Da] |
| 75 - 98 | max. 10 | < 300.000 | < 40.000 |
| 78 - 86 | 4 - 10 | < 160.000 | < 35.000 |
| 75 - 85 | 5 - 8 | < 150.000 | < 27.000 |
| 80 - 98 | | < 100.000 | 10.000 - 35.000 |
| 80 - 85 | | 20.000 - 300.000 | 10.000 - 27.000 |
| 82 - 84 | | 50.000 - 160.000 | |
| 92 - 98 | | 50.000 - 120.000 | |

So ist beispielsweise eine Zubereitung enthaltend GLYCOPYRROLAT und Chitosane mit A = 80 - 98%, B = 5 - 10 mPas, C = 50.000 - 120.000 Da und D = 10.000 - 27.000 Da ebenso bevorzugt für eine spezielle Deo/AT-Produktformulierung wie eine Zubereitung enthaltend Chitosane mit A = 82 - 84%, B = 5 - 8 mPas, C < 160.000 Da und D < 35.000 Da.

Überraschenderweise wurde gefunden, dass die Kombination von Glycopyrrolat und Chitosan und Zubereitungen, diese Kombination enthaltend, die Adhäsion, d.h. das Vermögen der Mikroorganismen an Oberflächen anzuhaften, herabsetzen, so dass sich deren übliche Anzahl auf solche Flächen verringert, oder auch, dass sich keine oder keine wesentlichen Mengen von Mikroorganismen mehr nachweisen lassen.

In einem Adhäsionsassay mit den Mikroorganismen *Corynebacterium jeikeium (C. jeikeium)* wurde die synergistische Verringerung der Adhäsion eindrucksvoll bestätigt, wie in Abbildung 1 dargestellt.

Eine Kombination von 0,1 Gew.% Glycopyrrolat und 0,025 bzw. 0,01 Gew.% Chitosan verringert die Adhäsion des Bakteriums C. jeikeium um mehr als 50% im Vergleich zum Einsatz von Chitosan gleichen Anteils ohne Glycopyrrolat-Zusatz.

Die Verwendung von Glycopyrrolat in Kombination mit Chitosan, insbesondere zu Anteilen von 0,1 Gew.% Glycopyrrolat und 0,01 bis 0,025 Gew.% Chitosan, ist somit prädestiniert.

Polyacrylate sind ebenfalls vorteilhaft im Sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

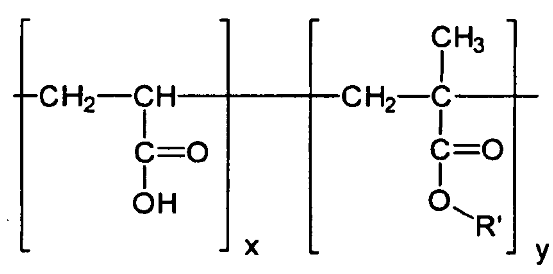

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F. Goodrich Company erhältlich sind, bevorzugt Polyacrylate aus der Gruppe der Carbopole der Typen 980, 981, 1382, 2984, 5984 sowie besonders bevorzugt Carbomer 2001

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/$C_{10-30}$Alkyl Acrylate Crosspolymer" tragen. Insbesondere vorteilhaft sind die unter den Handelsbezeichnungen Pemulen® TR1 und Pemulen® TR2 bei der B. F. Goodrich Company erhältlichen.

Vorteilhaft sind Verbindungen, die die INCI-Bezeichnung AmmoniumacryloyldimethyltaurateNinylpyrrolidoncopolymere tragen.

Erfindungsgemäß vorteilhaft weisen das oder die AmmoniumacryloyldimethyltaurateNinylpyrrolidoncopolymere die Summenformel $[C_7H_{16}N_2SO_4]$, $[C_6H_9NO]_m$ auf, einer statistischen Struktur wie folgt entsprechend

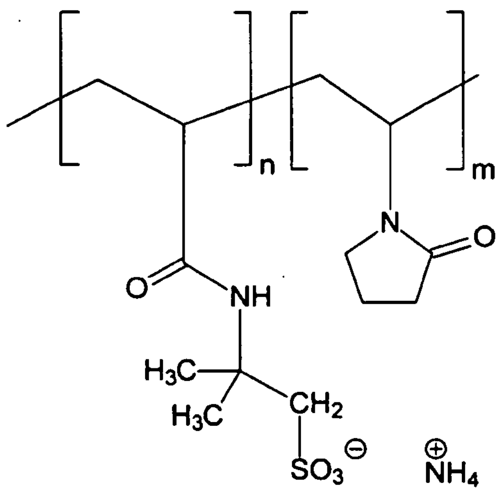

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel® EG oder Simugel® EG von der Gesellschaft Seppic S.A.

Weitere erfindungsgemäß vorteilhaft zu verwendende Hydrokolloide sind auch in Wasser lösliche oder dispergierbare anionische Polyurethane, welche vorteilhaft erhältlich sind aus

i) mindestens einer Verbindung, die zwei oder mehrere aktive Wasserstoffatome pro Moleküle enthält,
ii) mindestens einem Säure- oder Salzgruppen enthaltenden Diol und
iii) mindestens einem Diisocyanat.

Bei der Komponente i) handelt es sich insbesondere um Diole, Aminoalkohole, Diamine, Polyesterole und Polyetherole mit einem zahlenmittleren Molekulargewicht von jeweils bis zu 3000 oder deren Mischungen, wobei bis zu 3 Mol% der genannten Verbindungen durch Triole oder Triamine ersetzt sein können. Insbesondere umfasst die Komponente (i) mindestens 50 Gew.% eines Polyesterdiols, bezogen auf das Gesamtgewicht der Komponente (i). Als Polyesterdiole kommen alle diejenigen in Betracht, die üblicherweise zur Herstellung von Polyurethanen eingesetzt werden, insbesondere Umsetzungsprodukte aus Phthalsäure und Diethylenglycol, Isophthalsäure und 1,4-Butandiol, Isophthalsäure/Adipinsäure und 1,6-Hexandiol sowie Adipinsäure und Ethylenglycol oder 5-$NaSO_3$-Isophthalsäure, Phthalsäure, Adipinsäure und 1,6-Hexandiol.

Brauchbare Diole sind z.B. Ethylenglycol, Propylenglycol, Butylenglycol, Neopentylglycol, Polyetherole, wie Polyethylenglycole mit Molekulargewichten bis zu 3000, Blockcopolymerisate aus Ethylenoxid und Propylenoxid mit zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Bevorzugt sind Ethylenglycol, Neopentylglycol, Di-, Tri-, Tetra-, Penta- oder Hexaethylenglyol. Brauchbare Diole sind außerdem Poly(hydroxycarbonsäure)diole.

Geeignete Aminoalkohole sind z.B. 2-Aminoethanol, 2-(N-Methylamino)ethanol, 3-Aminopropanol oder 4-Aminobutanol.

Geeignete Diamine sind z.B. Ethylendiamin, Propylendiamin, 1,4-Diaminobutan und 1,6-Diaminohexan sowie $\alpha,\omega$-Diamine, die durch Aminierung von Polyalkylenoxiden mit Ammoniak herstellbar sind.

Bei der Komponente ii) handelt es sich insbesondere um Dimethylolpropansäure oder Verbindungen der Formeln

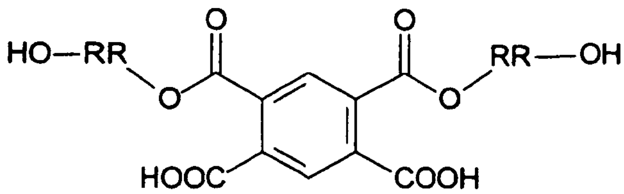

bzw.

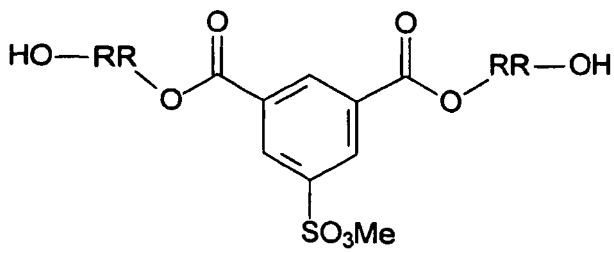

worin RR jeweils für eine $C_2$-$C_{18}$-Alkylengruppe steht und Me für Na oder K steht.

Bei der Komponente iii) handelt es sich insbesondere um Hexamethylendiisocyanat, Isophorondiisocyanat, Methyldiphenylisocyanat (MDI) und/oder Toluylendiisocyanat.

Die Polyurethane sind dadurch erhältlich, dass man die Verbindungen der Gruppen i) und ii) unter einer Inertgasatmosphäre in einem inerten Lösemittel bei Temperaturen von 70 bis 130°C mit den Verbindungen der Gruppe iii) umsetzt. Diese Umsetzung kann gegebenenfalls in Gegenwarte von Kettenverlängerern durchgeführt werden, um Polyurethane mit höheren Molekulargewichten herzustellen. Wie bei der Herstellung von Polyurethanen üblich, werden die Komponenten [(i)+(ii)]:(iii) vorteilhaft im molaren Verhältnis von 0,8 - 1,1 : 1 eingesetzt. Die Säurezahl der Polyurethane wird von der Zusammensetzung und der Konzentration der Verbindungen der Komponente (ii) in der Mischung aus den Komponenten (i)+(ii) bestimmt.

Die Säuregruppen enthaltenden Polyurethane sind nach Neutralisation (teilweise oder vollständig) wasserlöslich bzw. ohne Zuhilfenahme von Emulgatoren dispergierbar. In aller Regel weisen die Salze der Polyurethane eine bessere Wasserlöslichkeit oder Dispergierbarkeit in Wasser auf als die nicht neuralisierten Polyurethane. Als Base für die Neutralisation der Polyurethane können Alkalimetallbasen wie Natronlauge, Kalilauge; Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak und Amine verwendet werden. Besonders haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 - 40 % oder vollständig, d.h. zu 100 % erfolgen.

Diese Polymere und ihre Herstellung sind in DE-A-42 25 045 näher beschrieben, auf die hiermit in vollem Umfang Bezug genommen wird.

In Wasser lösliche oder dispergierbare, kationische Polyurethane und Polyharnstoffe sind aus

a) mindestens einem Diisocyanat, welches bereits vorher mit einer oder mehreren Verbindungen, die zwei oder mehrere aktive Wasserstoffatome pro Moleküle enthalten, umgesetzt worden sein kann, und
b) mindestens einem Diol, primären oder sekundären Aminoalkohol, primären oder sekundärem Diamin oder primären oder sekundären Triamin mit einem oder mehreren tertiären, quaternären oder protonierten tertiären Aminostickstoffatomen, herstellbar.

Bevorzugte Diisocyanate sind wie oben angegebene. Verbindungen mit zwei oder mehreren aktiven Wasserstoffatomen sind Diole, Aminoalkohole, Diamine, Polyesterole. Polyamiddiamine und Polyetherole.

Die Herstellung der Polyurethane erfolgt wie oben beschrieben. Geladene kationische Gruppierungen lassen

sich aus den vorliegenden tertiären Aminostickstoffatomen entweder durch Protonierung, z.B. mit Carbonsäuren wie Milchsäure oder durch Quaternisierung, z.B. mit Alkylierungsmitteln wie $C_1$- bis $C_4$-Alkylhalogeniden oder -sulfaten in den Polyharnstoffen erzeugen. Beispiele solcher Alkylierungsmittel sind Ethylchlorid, Ethylbromid, Methylchlorid, Methylbromid, Dimethylsulfat und Diethylsulfat.

Diese Polymere und ihre Herstellung sind in der DE-A-42 41 118 näher beschrieben, auf die hiermit in vollem Umfang Bezug genommen wird.

Lineare Polyurethane mit Carboxylatgruppen sind darstellbar aus

i) einer 2,2-Hydroxymethyl-substituierten Carbonsäure der Formel

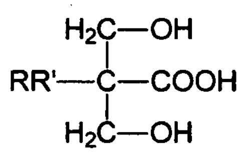

worin RR' für ein Wasserstoffatom oder eine $C_1$-$C_{20}$-Alkylgruppe steht, die in einer Menge verwendet werden, welche ausreicht, dass in dem Polyurethan 0,35- 2,25 milliäquivalente Carboxylgruppen pro g Polyurethan vorhanden sind,

ii) 10 - 90 Gew.%, bezogen auf das Gewicht des Polyurethans, einer oder mehrerer organischer Verbindungen mit nicht mehr als zwei aktiven Wasserstoffatomen und

iii) einem oder mehreren organischen Diisocyanaten.

Die im Polyurethan enthaltenden Carboxylgruppen werden abschließend mit einer geeigneten Base zumindest teilweise neutralisiert. Diese Polymere und ihre Herstellung sind in der EP-A-619 111 beschrieben, auf die hiermit in vollem Umfang Bezug genommen wird.

Carboxylhaltige Polykondensationsprodukte aus Anhydriden von Tri- oder Tetracarbonsäuren und Diolen, Diaminen oder Aminoalkoholen (Polyester, Polyamide oder Polyesteramide) sind weiterhin bevorzugt. Diese Polymere und ihre Herstellung sind in der DE-A-42 24 761 näher beschrieben, auf die hiermit in vollem Umfang Bezug genommen wird.

Polyacrylate und Polymethacrylate, wie sie in den DE-A-43 14 305, 36 27 970 und 29 17 504 näher beschrieben sind, werden ebenfalls bevorzugt. Auf diese Publikationen wird hiermit in vollem Umfang Bezug genommen.

Die erfindungsgemäß zur Anwendung kommenden Polymere besitzen vorzugsweise einen K-Wert von 25 - 100, bevorzugt 25 - 50. Die Polymere sind in dem erfindungsgemäßen Mittel im allgemeinen in einer Menge im Bereich von 0,2 - 20 Gew.%, bezogen auf das Gesamtgewicht des Mittels, enthalten. Das Salz kommt in einer zur Verbesserung der Austauschbarkeit der Polymere wirksamen Menge zur Anwendung. Im allgemeinen setzt man das Salz in einer Menge von 0,02 - 10 Gew.%, vorzugsweise 0,05 - 5 Gew.% und insbesondere 0,1 - 3 Gew.%, bezogen auf das Gesamtgewicht des Mittels, ein.

Es ist erfindungsgemäß vorteilhaft, das molare Verhältnis von Glycopyrroniumbromid zu einem oder mehreren Hydrokolloiden aus dem Bereich von 100 : 1 bis 1 : 100 , bevorzugt 50 : 1 bis 1 : 50, insbesondere bevorzugt 20 : 1 bis 1 : 20 zu wählen.

Gut geeignete Mono-, Oligo- und/oder Polysaccharide oder "Kohlenhydrat-Derivate", die sprachlich kurzgefasst auch unter die Bezeichnung "Kohlenhydrate" fallen sollen, sind Zucker und substituierte Zucker oder Zuckerreste enthaltende Verbindungen. Zu den Zuckern zählen insbesondere auch jeweils die Desoxy-Formen.

Bevorzugt werden die folgenden Wirkstoffe mit Zuckerstrukturen:

1. Monosaccharide

Gut geeignete Monosaccharide sind z.B. Tetrosen, Pentosen, Hexosen und Heptosen. Bevorzugt werden Pentosen und Hexosen. Die Ringstrukturen umfassen Furanosen und Pyranosen, umfasst sind sowohl D- als auch L-Isomere, ebenso wie α- und β-Anomere. Geeignet sind auch die Desoxy-Formen.

2. Disaccharide

Gut geeignete Disaccharide sind z.B. die durch binäre Verknüpfungen obiger Monosaccharide gebildeten Disaccharide. Verknüpfung kann als α- oder β-glycosidische Bindung zwischen den beiden Untereinheiten erfolgen. Saccharose, Maltose, Lactobiose werden bevorzugt.

3. Oligosaccharide

Gut geeignete Oligosaccharide bestehen aus mehreren, z.B. 2-7 Zuckereinheiten, vorzugsweise der unter 1 und 2 beschriebenen Zucker, insbesondere aus 2 bis 4 Einheiten in den bekannten, durch Kondensation entstandenen Bindungsformen und wie vorstehend genannt. Besonders bevorzugte Oligosaccharide sind neben den Disacchariden die Trisaccharide.

4. Aminozucker

Gut geeignet sind Mono-, Di- und Oligosaccharide, insbesondere wie vorstehend beschrieben, mit einer oder mehreren Aminogruppen, die acyliert, insbesondere acetyliert sein können. Bevorzugt werden Ribosylamin, N-Acetylglucosamin und N-Galactosylamin.

Weiterhin werden Zuckerester von organischen oder anorganischen Säuren vorteilhaft verwendet, beispielsweise Zuckerphosphate, Zuckerester mit Carbonsäuren oder sulfatierte Zucker, insbesondere Ester der vorstehend beschriebenen Zucker.

5. Bevorzugte Zuckerester der Phosphorsäure sind Glucose-1-phosphat, Fructose-1-phosphat, Glucose-6-phosphat oder Mannose-6-phosphat.

6. Bevorzugte Ester aus Zuckern und Carbonsäuren werden mit Carbonsäuren der Kettenlänge C1 bis C24, z.B. erhalten, zum Beispiel Cetearylglucosid (Fa. Seppic: Montanol 68); Caprylyl/Caprylglucosid (Fa. Seppic: Oramix CG-110); Decylglucosid (Fa. Seppic: Oramix NS-10), insbesondere aber auch die Zuckeracetate, bevorzugt der vorstehenden Zucker.

7. Bevorzugt werden auch die Zuckerether aus Zuckern, insbesondere der vorstehenden Zucker, mit Alkoholen der Kettenlänge C1 bis C24, z.B. PlantarenR 1200 (Fa. Henkel) oder PlantarenR 2000 (Fa. Henkel).

8. Weiterhin sind z.B. die Umsetzungsprodukte von Zuckern mit Ethylenoxid und/oder Propylenoxid geeignet, vorzugsweise mit den vorstehenden Zuckern. Geeignet sind E/O-Grade von einer bis 40 Ethereinheiten.

9. Glykolipide

Bevorzugte Glykolipide sind Glykosphingolipide, insbesondere Ceramide, Cerebroside, Ganglioside und Sulfatide.

10. Polysaccharide (natürlichen und synthetischen Ursprunges)

Die Polysaccharide können unverzweigt oder verzweigt sein und es sind sowohl die Homopolysaccharide als auch die Hetero-Polysaccharide, jeweils insbesondere mit solchen Zuckern, wie unter 1. bis 7. beschrieben, geeignet. Bevorzugte Polysaccharide sind Stärke, Glykogen, Cellulose, Dextran, Tunicin, Inulin, Chitin, insbesondere Chitosane, Alginsäure und Alginate, Pflanzen-Gumme, Körperschleime, Pektine, Mannane, Galactane, Xylane, Araban, Polyosen, Chondroitinsulfate, Heparin, Hyaluronsäure und Glycosaminoglykane, Hemicellulosen, substituierte Cellulose und substituierte Stärke, insbesondere jeweils die hydroxyalkylsubstituierten Polysaccharide.

Besonders geeignet ist Chitosan, wie zuvor dargestellt.

Die Polysaccharide können z.B. aus 4 bis 1.000.000, insbesondere 10 bis 100.000 Monosacchariden bestehen. Vorzugsweise werden jeweils solche Kettenlängen gewählt, die gewährleisten, dass der Wirkstoff in der jeweiligen Zubereitung löslich oder in sie einzuarbeiten ist.

Die erfindungsgemäßen zusätzlichen Aktivstoffe können einzeln eingesetzt werden. Es ist aber auch möglich, zwei, drei oder auch mehrere Aktivstoffe zusammen zu verwenden. Insbesondere können Monosaccharide und Oligosaccharide kombiniert werden, wobei jeweils ein Saccharid, aber auch zwei oder drei oder mehrere Zucker gewählt werden können. Zusammen mit den vorstehend genannten Zuckern oder deren Kombinationen können vorteilhaft ein Polysaccharid oder auch mehrere Polysaccharide verwendet werden.

Bevorzugt werden die folgenden Kombinationen und Zubereitungen damit und deren Verwendungen. Bevorzugt werden Wirkstoffkombinationen mit mindestens drei Wirkstoffen, ausgewählt aus der Gruppe enthaltend

Aldopentosen und Ketopentosen und
Aldohexosen und Ketohexosen und
Aldoheptosen und Ketoheptosen.

Die genannten Zucker können insbesondere auch in ihrer Desoxy-Form und insbesondere auch in der Form der erfindungsgemäßen Derivate vorliegen. Dies gilt auch für die folgenden bevorzugten Kombinationen.

Besonders bevorzugt werden Kombinationen, insbesondere Kombinationen von mindestens drei Wirkstoffen, die mindestens einen Desoxy-Zucker oder mindestens ein Desoxy-Zucker-Derivat oder mindestens ein Disaccharid oder mindestens ein Trisaccarid enthalten, wobei diese auch jeweils in der Form der erfindungsgemäßen Derivate oder auch in der Desoxy-Form vorliegen können.

Weiterhin werden Kombinationen, insbesondere Kombinationen von mindestens drei Wirkstoffen bevorzugt, die Fucose enthalten, wobei diese auch jeweils in der Form der erfindungsgemäßen Derivate vorliegen kann.

Besonders bevorzugt werden die folgenden Wirkstoffkombinationen a) - f):

a) Fucose,
Raffinose und
Galactose

b) Glucose-6-phosphat,
Mannose-6-phosphat und
Mannose

c) Raffinose,
N-Acetyl-glucosamin, und
Fucose

d) Mannose,
Rhamnose und
Fucose

e) Galactose,
N-Acetyl-glucosamin und
Fucose

f) Mannose,
Raffinose und
Galactose.

Bevorzugt werden auch die jeweiligen Einzelkomponenten der Kombinationen und die mit jeweils zwei Komponenten zu bildenden Zweier-Kombinationen aus den drei Wirkstoffen jeweils einer Dreier-Kombination.

Vorteilhaft können auch jeweils mit einem Zucker oder mehreren Zuckern aus der Gruppe der Monosaccharide und/oder der Oligosaccharide ein oder mehrere Zucker aus der Gruppe der Zuckerphosphate und/oder der Aminozucker und Acetylaminozucker kombiniert werden.

Es ist erfindungsgemäß vorteilhaft, das molare Verhältnis von Glycopyrroniumbromid zu Mono-, Oligo- und/ oder Polysacchariden aus dem Bereich von 100 : 1 bis 1 : 100 , bevorzugt 50 : 1 bis 1 : 50, insbesondere bevorzugt 20 : 1 bis 1 : 20 zu wählen.

Es war erstaunlich, dass die erfindungsgemäßen Zusammensetzungen nicht nur für kosmetische Zwecke geeignet sind, sondern überdies wirkungsvoller und schonender sind als die Zusammensetzungen des Standes der Technik. Es scheint, dass die Silikate die Sedimentation des Glycopyrroniumbromids in der Ölphase von ölhaltigen Zubereitungen verhindern, zumindest aber in signifikanter Weise vermindern. Der Zusatz von Silikaten steigert die antitranspirierende Wirkung von Glycopyrroniumbromid in überraschender und nicht vorhersehbarer Weise.

Als Silikate werden vorzugsweise modifizierte Schichtsilikate, Tonmineralien und/oder Kieselsäuren verstanden.

Silikate sind Salze und Ester (Kieselsäureester) der Orthokieselsäure [$Si(OH)_{4]}$ und deren Kondensationsprodukte. Chemische Formeln lassen sich für Schichtsilikate nur angenähert aufstellen, da sie ein großes Ionenaustausch-Vermögen besitzen und Silizium gegen Aluminium und dieses wiederum gegen Magnesium, $Fe^{2+}$-, $Fe^{3+}$, $Zn^{2+}$ und dergleichen ausgetauscht werden kann. Die daraus möglicherweise resultierende negative Ladung der Schichten wird in der Regel durch Kationen, insbesondere durch $Na^+$ und $Ca^{2+}$ in Zwischenschicht-Positionen ausgeglichen.

Vorteilhafte Schichtsilikate sind beispielsweise solche, deren größte Ausdehnungsrichtung im unmodifizierten und ungequollenen Zustand im Mittel eine Länge von weniger als 10 $\mu$m hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung

hängt selbstverständlich von der Menge an eingelagerten Substanzen ab.

Vorteilhafte modifizierte Schichtsilikate im Sinne der vorliegenden Erfindung sind beispielsweise modifizierte Smektite (Smectite). Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus $[(Si,Al)O_4]$-Tetraedern umgeben ist. Vorteilhafte modifizierte Smektite sind z. B. modifizierte Montmorillonite. Montmorillonite werden durch die angenäherte chemische Formel $Al_2[(OH)_2/Si_4O_{10}]\cdot n\ H_2O$ bzw.

$Al_2O_3 - 4\ SiO_2\cdot H_2O\cdot n\ H_2O$ beschrieben und stellen zu den dioktaedrischen Smektiten gehörende Tonmineralien dar.

Besonders vorteilhaft im Sinne der vorliegenden Erfindung sind ferner beispielsweise modifizierte Hektorite. Hektorite gehören zu den Smektiten und haben die angenäherte chemische Formel $M^+_{0.3}(Mg_{2.7}Li_{0.3})[Si_4O_{10}(OH)_2]$, worin $M^+$ meist $Na^+$ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die "Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quaternären Ammoniumverbindungen - erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hektoriten, die durch Ionen austausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden:

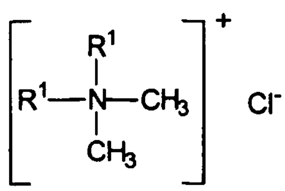

worin die Reste R1 unabhängig voneinander gewählt werden aus hydrierten Talgresten mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind. Ebenso bevorzugt ist erfindungsgemäß Quaternium-90 Bentonite, ein Reaktionsprodukt aus Bentonit und Quaternium-90, das von der Firma Süd-Chemie unter dem Handelsnamen Tixogel VP-V zu beziehen ist. Die Bezeichnung deutet daraufhin, dass die Alkylreste R1 bei diesem Produkt pflanzlichen Ursprungs sind, wodurch sich besonders vorteilhafte Eigenschaften im Sinne der vorliegenden Erfindung bzgl. Verdickung der Matrixphase und Wiederaufschüttelbarkeit des suspendierten Antitranspirant-Wirkstoffes ergeben.

Bei der Verwendung von Tonmineralien kann zusätzlich ein sogenannter Aktivator verwendet werden. Diesem kommt die Aufgabe zu, das eingesetzte Tonmineral zu delaminieren, was auch als Aktivierung bezeichnet wird. Üblicherweise werden hierzu kleine, polare Moleküle wie Propylenglycolcarbonat und Ethanol eingesetzt, die sich unter mechanischem Energieeintrag zwischen die Schichten der Tonminerallamellen schieben und somit den gewünschten Vorgang durch elektrostatische Wechselwirkung mit diesen ermöglichen. Darüber hinaus bilden sie Wasserstoffbrückenbindungen zu den delaminierten Tonmineralplättchen aus und sorgen durch diese Brückenfunktion - in einer Doppelfunktion quasi als Klammer und Scharnier - für den Zusammenhalt der entstehenden spielkartenhausähnlichen Struktur.

Die beschriebenen Vorgänge auf mikroskopischer Ebene bewirken eine Verdickung der flüssigen Matrix und spiegeln sich in einer makroskopisch leicht beobachtbaren Viskositätserhöhung des Systems wieder. Typischerweise zeigen diese Systeme eine stark ausgeprägte Thixotropie.

Kieselsäuren sind Verbindungen der allgemeinen Formel $(SiO_2)m\cdot nH_2O$. Erfindungsgemäß haben die pyrogenen Kieselsäuren eine große Bedeutung. Unter der Bezeichnung pyrogene Kieselsäuren werden hochdisperse Kieselsäuren zusammengefasst, die durch Flammenhydrolyse (Typ A) hergestellt werden. Dabei wird Siliciumtetrachlorid in einer KnallgasFlamme zersetzt:

$$H_2 + O_2 + SiCl_4 \xrightarrow{1000^\circ C} SiO_2 + 4HCl.$$

Sie besitzen an ihrer nahezu porenfreien Oberfläche deutlich weniger OH-Gruppen als Fällungs-Kieselsäuren. Wegen ihrer durch die Silanol-Gruppen bedingten Hydrophilie werden die synthetischen Kieselsäuren häufig einem chemischen Nachbehandlungsverfahren unterzogen, bei denen die OH-Gruppen z. B. mit organischen Chlorsilanen reagieren. Dadurch entstehen modifizierte, z. B. hydrophobe Oberflächen, welche die anwendungstechnischen Eigenschaften der Kieselsäuren wesentlich erweitern. Sie sind unter den Handelnamen Aerosil und Cab-O-Sil mit verschiedenen Eigenschaften erhältlich.

Es ist zwar bekannt, Silikate, auch modifizierte Tone wie Bentonite, in kosmetischen Formulierungen einzusetzen. Bekannt sind ferner kosmetische Desodorantien mit einem Gehalt an solchen modifizierten Tonen, z.B. EP-0 319 168. Diese Stoffe indes sind gemäß ihrer chemischen Natur völlig ungeeignet, als desodorierende Agenzien zu wirken. Sie stellen in den Formulierungen des Standes der Technik nur Hilfs- oder Zusatzstoffe dar, die die Konsistenz der Formulierungen verbessern sollen oder ähnliches.

Es hat sich herausgestellt, dass sich Silikate erfolgreich in alle gewöhnlichen Formulierungstypen von Desodorantien einarbeiten lassen, beispielsweise in Aerosole, Puder, Pumpsprays, Pudersprays, Roll-Ons, Deostifte, Tinkturen und weitere mehr.

Als besonders vorteilhaft haben sich Formulierungen mit einem wirksamen Gehalt an Bentoniten, Smektiten, sowie Kieselsäuren herausgestellt.

Es kann ferner von Vorteil sein, den Zusammensetzungen die üblichen kosmetischen Zusatzstoffe einzuverleiben, beispielsweise Konservierungsstoffe, Antioxidantien, Photostabilisatoren usw.

Ansonsten sind die üblichen Maßregeln für das Zusammenstellen von kosmetischen Formulierungen zu beachten, die dem Fachmann geläufig sind.

Die Silikate können auf einfache Weise in die erfindungsgemäßen Zusammensetzungen eingearbeitet werden. Bevorzugt werden sie als feinstzerteilte Partikel den übrigen Bestandteilen der Formulierungen zugesetzt, vorteilhaft in Gegenwart eines Dispergiermittels.

Es ist erfindungsgemäß vorteilhaft, das molare Verhältnis von Glycopyrroniumbromid zu einem oder mehreren Silicaten aus dem Bereich von 100 : 1 bis 1 : 100 , bevorzugt 50 : 1 bis 1 : 50, insbesondere bevorzugt 20 : 1 bis 1 : 20 zu wählen.

Vorzugsweise liegen die Silicate in Konzentrationen von 0,05 -10,00 Gew.% vor. Besonders bevorzugt liegen die Silicate in Konzentrationen von 0,10 - 7,50 Gew.%, ganz besonders bevorzugt in Konzentrationen von 0,20 - 5,00 Gew.% vor. Die Konzentrationsangaben beziehen sich jeweils auf das Gesamtgewicht der Zusammensetzung.

Die Kombination von Glycopyrrolat mit Parfumstoffen gewählt aus der Gruppe der Aldehyde, Ester, Jonone, Methyljonone, Damascone, Salicylate, Acetale und/oder Holzkörper (Iso E Super) führt zu einer verbesserten desodorierenden Wirkung.

Um die negativen Effekte des Schwitzens zu minimieren wird durch den Einsatz von AT-Mitteln gemeinhin die Schweißproduktion der Achsel reduziert und mit Deowirkern die Entstehung unangenehmen Achselgeruchs verhindert. Entscheidend für die Wahrnehmung der Produktleistung durch den Verbraucher ist dabei die Kombination mit einem Parfum, das einen dauerhaft angenehmen Geruch der Achsel sichert. Bisher können hier bestimmte Riechstoffgruppen, die für ein optimales Parfüm notwendig wären, nicht oder nur in unbedeutenden Mengen eingesetzt werden, da sie in Gegenwart von herkömmlichen AT-Mitteln nicht stabil eingesetzt werden können.

Überraschend wurde gefunden, dass beim Austausch von klassischen, Aluminium-basierten AT-Wirkern gegen Glycopyrrolat Parfüminhaltsstoffe stabil eingearbeitet werden können, die zwar wichtig für die Optimierung des Parfums sind, bisher aber für die prominente Verwendung ausgeschlossen waren. Zu diesen prominenten Parfumstoffen zählen insbesondere die chemischen Gruppen der Aldehyde, Ester, Jonone und Methyljonone, Damascone, Salicylate, Acetale und viele Holzkörper, speziell Iso E Super.

Es ist insbesondere bevorzugt Glycopyrrolat in Kombination mit Parfumstoffen gewählt aus der Gruppe der Aldehyde, hier bevorzugt Lilial, Hexylzimtaldehyd-Alpha, aber auch Helional, Citral, Vertocitral, Phenylacetaldehyd, Ester, bevorzugt Linalylacetat, Ter-Pinylacetat, aber auch Dimethylbenzylcarbinylbutyrat, Allylcaprylat, Jonone, Methyljonone, Damascone, Salicylate, Acetale und/oder Holzkörper, bevorzugt Iso E Super, aber auch Vertofix Coeur, Trimofix O, in kosmetischen Zubereitungen als Antitranspirant oder Deodorant einzusetzen. Glycopyrrolat anstelle der sonst üblichen AT-Wirker wie Al-Salze einzusetzen ermöglicht darüber hinaus die stabile Einarbeitung von Parfuminhaltstoffen. Es ist daher bevorzugt auf den Einsatz von Aluminium-basierenden AT-Wirkern zu verzichten und dennoch eine AT- bzw. Desodorantwirkung zu erzielen.

Bevorzugt ist der Einsatz von Glycopyrrolat an Stelle von beispielsweise Aluminiumchlorohydrat (ACH) in Kombination mit einem oder mehreren Parfumstoffen zu einem Anteil von < 1 Gew.%, insbesondere bis zu 0,5 Gew. %, bezogen auf die Gesamtmasse der kosmetischen Zubereitung.

Die aufgeführten Parfuminhaltsstoffe sind bisher für die prominente Verwendung ausgeschlossen und nicht oder nur in unbedeutenden Mengen enthalten, da sie in Gegenwart klassischer AT-Mittel nicht stabil sind. Durch den erfindungsgemäßen Einsatz dieser Parfumrohstoffen in Deo/AT-Produkten ist auch längere Zeit nach Auftragen immer noch der Geruch des Parfüms in der Achsel wahrnehmbar, wodurch eine zusätzliche desodorierende Wirkung erreicht wird.

Die Kombination von Glycopyrrolat mit Phenoxyethanol bewirkt eine Deo/AT-Wirkungsteigerung. Das hydrophile Glycopyrrolat in Kombination mit dem lipophilen Phenoxyethanol führt dazu, dass Glycopyrrolat sich in der Ölphase einer kosmetischen Zubereitung, beispielsweise einer Emulsion, aufhält. Der lipophilen Hautcharakters hat dadurch eine bessere Hauteindringung des Glycopyrrolats zur Folge, was wiederum eine gesteigerte Wirkleistung zur Folge hat.

Die Kombination Glycopyrrolat mit antimikrobiell wirksamem Glas entsprechender Zusammensetzung ermöglicht die Bereitstellung kosmetischer Zubereitungen, die neben der AT- und antimikrobiellen Wirkung zudem ästhetisch ansprechend und vor allem hautpflegend sind.

Besonderer Vorteil der erfindungsgemäßen Zubereitungen ist, dass die Silbergläser, insbesondere auch durch Ihre vorteilhaften Feinverteilung, zu keinerlei Einbußen in der Anwendung im Vergleich zu gängigen Kosmetika führen. Der erfindungsgemäße Vorteil liegt in der Lagerstabilität und damit Wirksamkeit über einen längeren Zeitraum und insbesondere in der Verfärbungsstabilität gegenüber äußeren Einflüssen wie Wärme oder Sonnenlicht. Die erfindungsgemäßen Zubereitungen weisen damit gegenüber anderen silberhaltigen Kosmetika keine Schwarz- oder Dunkelfärbung auf.

Die Angabe der Glaszusammensetzung in mol% gibt die Bestandteile ohne Silberoxid an. Zusätzlich ist die Silberoxidmenge in Gew.%, bezogen auf die Gesamtmasse des dann silberhaltigen Glases angegeben.

Besonders ein Material mit einer Glaszusammensetzung

| | | |
|---|---|---|
| $P_2O_5$ | 40 - 60 | mol%, |
| $R^1O$ | 35 - 55 | mol%, |
| $R^2{}_2O$ | 0 - 5 | mol%, |
| $SiO_2$, $Al_2O_3$ | 5-20 | mol% und |
| $Ag_2O$ | 0.1 -5 | Gew.%. und |

ein Material mit einer Glaszusammensetzung

| | | |
|---|---|---|
| $P_2O_5$ | 45-55 | mol%, |
| CaO, MgO | 35-50 | mol%, |
| $Na_2O$ $K_2O$ | 0-5 | mol%, |
| $SiO_2$ | 0-5 | mol%, |
| $Al_2O_3$ | 5-15 | mol% und |
| $Ag_2O$ | 0,5 - 3 Gew.% | |

sowie bevorzugt ein Material mit einer Glaszusammensetzung

| | | |
|---|---|---|
| $P_2O_5$ | 50 | mol%, |
| MgO | 44 | mol%, |
| $Al_2O_3$ | 6 | mol%, |

bezogen auf die Gesamtmenge des silberoxidfreien Glases und

| | | |
|---|---|---|
| $Ag_2O$ | 2 | Gew. %, |

bezogen auf die Gesamtmasse des Glases,
oder mit einer Glaszusammensetzung

| | | |
|---|---|---|
| $P_2O_5$ | 73,35 | Gew.%, |
| MgO | 18,33 | Gew.%, |
| $Al_2O_3$ | 6,32 | Gew.% und |
| $Ag_2O$ | 2,0 | Gew.%, |

bezogen auf die Glasgesamtmasse, hat sich als besonders anwendungsfreundlich und wirksam erwiesen.
Ionpure A ist ein bevorzugtes silberhaltiges Glas und Handelsprodukt der Firma Ishizuka, JP. Ebenso ist C 1193 ein auf dem Markt erhältliches silberhaltiges Glas.
In der Abbildung 3 ist die Reduktion der Zellzahl (C. xerosis) bei Zubereitungen umfassend GLYCOPYRROLAT und silberhaltige Gläser im Vergleich zu Zubereitungen umfassend nur GLYCOPYRROLAT bzw. silberhaltige Gläser (C-1193 bzw. Ionpure A) dargestellt. Auffallend auch hier der Synergismus im Hinblick auf die Zellzahlreduktion.

Als erfindungsgemäße Zubereitung bzw. Material wird in diesem Zusammenhang alles das verstanden, was auf die menschliche oder tierische Haut aufgetragen oder appliziert werden kann und die Auf- und Abgabe von Silber an die Umgebung gewährleisten kann. Diese Materialien sind insbesondere Kosmetika, insbesondere kosmetische Zubereitungen auf Emulsionsbasis, desinfizierende Reinigungszubereitungen sowie antimikrobiell wirksame Hautauflagen, Pads oder Tücher.

Diese Materialien enthalten bevorzugt 0,001 - 40 Gew.%, bevorzugt 0,05 - 1 Gew.% des silberhaltigen Glases, bezogen auf die Gesamtmasse des Materials.

Das hierbei enthaltene antimikrobiell oder desinfizierend wirksame Silber ist in Form freier Silberionen verfügbar und nur der Zusammensetzungsschreibweise des Glases entsprechend als Oxid $Ag_2O$ gekennzeichnet.

Bei den silberglasenthaltenden Zubereitungen handelt es sich in der Regel um Emulsionen oder wässrige Hydrogele, die neben üblichen Feuchthaltesubstanzen auch spezielle Wirkstoffe enthalten können, wie beispielsweise

- ■ entzündungslindernde und kühlende Stoffe,
- ■ lokal anästhesierende Stoffe und/oder
- ■ andere topisch anzuwendende kosmetische, pharmazeutische und/oder dermatologische Wirkstoffe.

Eingesetzt werden z. B. aus Pflanzen gewonnene entzündungslindernde bzw. -hemmende Wirkstoffe wie Azulen und Bisabolol (Kamille), Glycyrrhizin (Süßholzwurzel), Hamamelin (Hamamelis) oder Gesamtextrakte, z. B. aus Aloe vera oder Kamille. Diese zeigen bei leichteren entzündlichen Formen und lokal begrenzten Erythemreaktionen gute Erfolge. Gleiches gilt für Cremes mit einem hohen Gehalt an ätherischen Ölen oder Panthenol.

Die Zubereitungen dienen aufgrund der antimikrobiellen bzw. desinfizierenden Wirkung des Silberglases u.a. zur Prophylaxe und Behandlung von entzündlichen Hautzuständen und/oder zum Hautschutz.

Es war überraschend und für den Fachmann nicht vorauszusehen, dass eine Wasser-in-Silikonöl-Basis für ein klares AT-Gel umfassend Glycopyrrolat als Wirkstoff die Aufgaben umfassend löst. Der komplette oder teilweise Ersatz von Aluminiumsalzen durch Glycopyrrolat ermöglicht, den Brechungsindex der wäßrigen Phase so zu reduzieren, dass in der Siikonölphase ein wesentlich größerer Anteil an preiswerteren Silikonölen mit niedrigerem Brechungsindex eingearbeitet werden kann. Hierdurch wird auch gleichzeitig die sensorisch unerwünschte Klebrigkeit der Produkte signifikant reduziert. Der Verzicht auf Aluminiumsalzen ist somit wie in den zuvor beschriebenen Ausführungsformen auch hier bevorzugt.

Durch die größere Auswahlmöglichkeit für die Silikonölphase kann gleichzeitig auch der Anteil der schwerflüchtigen Silikonöle erhöht werden, so dass die für den Verbraucher ungewünschte Eintrübung der Produkte nach dem Öffnen signifikant reduziert ist.

Der Ersatz von Aluminium- und Aluminiumzirkoniumsalzen durch Glycopyrrolat erleichtert außerdem die Herstellung transparenter Gele, da durch die Reduktion bzw. den Wegfall der galenisch häufig schwierig einzuarbeitenden Aluminiumsalze zusätzliche Hilfsstoffe zur Anpassung der Brechungsindices von wässriger Phase und Ölphase eingesetzt werden können. Speziell der Austausch von leichtflüchtigen Ölen gegen schwerer flüchtige Hilfststoffe und Öle verringert dabei das Risiko der Eintrübung des fertigen Gels durch Abdampfen leichtflüchtiger Formelbestandteile vor oder während des Mischens von wässriger und Ölphase.

Überraschend und für den Fachmann unerwartet zeigt sich außerdem, dass Glycopyrrolat im Gegensatz zu Standard-AT-Mitteln auch aus der inneren Phase heraus in der Lage ist, seine AT-Wirkung ohne Zeitverzögerung und ohne Leistungseinschränkung zu entfalten.

Ein weiterer sinnvoller Effekt der erfindungsgemäßen Gele ist, dass sich durch den Austausch der stark sauren Aluminiumsalze gegen Glycopyrroniumbromid eine bessere hautpflegende Eigenschaft einstellt.

Weiterer Vorteil ist, dass eine Ölphase mit erhöhtem Anteil an Silikonölen mit niedrigerem Brechungsindex zu verbesserter Sensorik führt.

Erfindungsgemäß sind Antitranspirantzubereitungen in transparenter Gelform auf Basis von O/W-PIT-Emulsionen mit einem Gehalt an Glycopyrroniumbromid bevorzugt.

Es war überraschend und für den Fachmann nicht vorauszusehen, dass eine mittels PIT-Technologie hergestellte Öl-in-Wasser-Basis für ein klares AT-Gel umfassend Glycopyrrolat als Wirkstoff die gestellten Aufgaben umfassend löst. Der Ersatz von Aluminiumsalzen durch Glycopyrrolat ermöglicht größere Freiheiten bei der Auswahl des Emulgatorsystems, z. B. auch den Einsatz PEG-freier Emulgatoren, die im Vergleich eine gesteigerte Hautverträglichkeit zeigen. Bedingt durch den Wegfall der stark kationischen Aluminiumsalze können außerdem geringere Mengen an

Assoziativverdickern eingesetzt werden, um zur gleichen Viskosität zu kommen wie bei ACH-haltigen Gelen mit höheren Gehalten an Assoziativverdickern. Außerdem stehen durch den Verzicht auf ACH weitere Assoziativverdicker zur Verfügung auf deren Einsatz aus Stabilitätsgründen ansonsten verzichtet werden muss.

Auf diese Weise erhält man klare AT-Gele, die auch nach Öffnen keine Trübung durch Abdampfen leichtflüchtiger Ölkomponenten zeigen und trotzdem in der Herstellung eine größere Formelvariabilität zu niedrigeren Kosten bieten als klassische, transparente PIT-Emulsionen.

Überraschend und für den Fachmann nicht zu erwarten war, dass durch den Austausch der Aluminiumsalze gegen Glycopyrrolat die PIT-Temperatur sich hin zu niedrigeren Temperaturen verschiebt. Dadurch ist es zum einen möglich, die Energiekosten bei der Herstellung zu reduzieren, zum anderen können noch kleinere Tröpchengrößen für die Ölphase eingestellt werden, so dass die Gele noch transparenter werden.

Ein weiterer sinnvoller Effekt der erfindungsgemäßen Gele ist, dass sich durch den Austausch der stark sauren Aluminiumsalze gegen Glycopyrroniumbromid eine bessere hautpflegende Eigenschaft einstellt.

Der Verzicht auf Aluminiumbasierende AT-wirkstoffe ist somit ebenso bevorzugt wie der Verzicht auf PEG, bzw. positiv ausgedrückt den Einsatz von PEG-freien Emulgatoren. Erfindungsgemäß werden Antitranspirantzubereitungen in Stiftform mit vorteilhafter Sensorik und reduziertem Weißeleffekt durch Kombination von wasserhaltigen Seifengelstift-Formeln mit einem Gehalt an Glycopyrroniumbromid erhalten.

Überraschend und für den Fachmann nicht zu erwarten konnte aus Seifegelstiften eine AT-Wirkung erzielt werden, ohne

- dass durch die Zugabe des AT-Mittels die Gelstruktur des Stifts negativ beeinflusst
- oder dass die AT-Wirkung des AT-Mittels durch den hohen pH-Wert (um 10) des Seifengelstifts negativ beeinflusst wurde.

Ein weiterer sinnvoller Effekt der erfindungsgemäßen Gele ist, dass sich durch den Austausch der stark sauren Aluminiumsalze gegen Glycopyrroniumbromid sich eine bessere hautpflegende Eigenschaft einstellt.

Überraschend wurde gefunden, dass Glycopyrrolat aus tensidhaltigen Reinigungsformulierungen auch nach der Reinigung der Haut eine Deo/AT-Wirkung entfaltet. Dies ist dadurch zu erklären, daß der Wirkmechanismus von Glycopyrrolat sich grundsätzlich von dem anderer AT-Wirker unterscheidet. Die Schweißdrüsen werden nicht verstopft, sondern inaktiviert.

Besonders vorteilhaft ist der Einsatz in flüssigen oder Stückseifen. In Kombination mit Seifen bilden die klassischen AT-Wirker unlösliche Niederschläge, die zu Verklumpungen führen. Mit Glycopyrrolat ist dies nicht der Fall.

Besonders vorteilhaft ist außerdem die Kombination von Glycopyrrolat mit kationischen Polymeren und/oder lipophilen Ölen in tensidhaltigen Produkten, da diese die Substantivität des Wirkstoffs und somit die Wirksamkeit deutlich verbessern.

Seifenherstellung:

Die Grundseifennudeln werden mit dem Farbslurry und den übrigen Komponenten in einen üblichen Seifenmischer (Schneckenmischer mit Lochsieb) dosiert, durch mehrmaliges Vermischen homogenisiert, über eine StranGlycopyrrolatresse ausgetragen, geschnitten und in üblicher Weise zu Stücken verarbeitet.

Demzufolge ist auch hier bevorzugt auf den Einsatz von AT-mitteln, insbesondere Aluminiumhaltigen Antitranspirantien, wie ACH, zu verzichten.

Die kosmetischen, pharmazeutischen oder dermatologischen Formulierungen wie zuvor dargestellt im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen, wenn wie zuvor dargestellt nicht in ihrer Form bevorzugt gewählt werden. Solche Emulsionen können vorzugsweise auch eine Mikroemulsion, eine Pickering-Emulsion oder eine sprühbare Emulsion sein. In diesem Falle könnten pflasterartige Applikationsformen mit ebendiesen Emulsionen getränkt werden.

Entsprechend der erfindungsgemäßen Verwendung sind die kosmetischen Zubereitungen, insbesondere Antitranspirantien bzw. Desodorantien, besonders vorteilhaft dadurch gekennzeichnet, dass das Glycopyrroniumbromid in Konzentrationen von 0,01 - 10,00 Gew.%, bevorzugt 0,05 - 5,00 Gew.%, besonders bevorzugt 0,1 - 3,00 Gew.%, jeweils bezogen auf das Gesamtgewicht der Zusammensetzung, vorliegt.

Entsprechend der erfindungsgemäßen Verwendung sind die Zubereitungen besonders vorteilhaft dadurch gekennzeichnet, dass der oder die Aktivststoffe jeweils unabhängig voneinander oder in Kombination zu einem Anteil von 0,01 - 10,00 Gew.%, bevorzugt 0,05 - 5,00 Gew.%, besonders bevorzugt 0,1 - 3,00 Gew.%, jeweils bezogen auf das Gesamtgewicht der Zusammensetzung, vorliegen.

Die Wirkstoffkombinationen lassen sich bevorzugt wählen indem die nachfolgenden Gewichtsverhältnisse eingestellt werden:

Es wird (A : B : C) wie a : b : c gewählt, wobei a, b und c unabhängig voneinander positive rationale Zahlen von 1 bis 200, bevorzugt von 1 bis 50 darstellen und

- A stellt dabei die der Konzentration von Glycopyrroniumbromid in Gewichtseinheiten (z.B. Gew.%) dar,
- B stellt dabei die Konzentration des Aktivstoffes, einer oder mehrerer dialkyl-substituierter Essigsäuren in denselben Gewichtseinheiten dar,
- C stellt dabei die Konzentration eines zweiten Aktivstoffes dar, gewählt aus der Gruppe der Stoffe a.) bis k.) in denselben Gewichtseinheiten, jeweils bezogen auf das Gesamtgewicht der Zubereitung.

Bevorzugt ist dann eine Wirkstoffkombination, bei denen der Quotient

$$(B + C) / A$$

aus dem Bereich zwischen 0,5 und 200, bevorzugt aus dem Bereich zwischen 1 und 50 gewählt wird.

Erfindungsgemäß hat sich herausgestellt, dass die gestellten Aufgaben, insbesondere einer gesteigerten antitranspirierenden und desodorierenden Wirkung, verbesserter hautpflegender Eigenschaften, besser als Vehikel für kosmetische und medizinisch-dermatologische Wirkstoffe dienend, und/oder besserer sensorischer Eigenschaften, wie beispielsweise Verteilbarkeit oder Einzugsvermögen in die Haut, erreicht werden, wenn Glycopyrrolat in Kombination mit mehreren Aktivstoffen und/oder formuliert in Form einer W/Si - Emulsion, eines O/W - Gels, einer W/O-Emulsion, eines Aerosols, eines wasserfreien Stifts oder Seifengelstifts bereit gestellt wird. Erfindungsgemäß können dabei bevorzugt mehrere oder alle zuvor beschriebenen Aktivstoffe in der kosmetischen Zubereitung enthalten sein.

Vorteilhaft können erfindungsgemäßen Zubereitungen auch übliche Desodorantien zugesetzt werden. Den üblichen kosmetischen Desodorantien liegen unterschiedliche Wirkprinzipien zugrunde. Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Menge der Desodorantien (eine oder mehrere Verbindungen) in den Zubereitungen beträgt vorzugsweise 0,01 bis 10 Gew.%, bevorzugt 0,05 bis 5 Gew.% bezogen auf das Gesamtgewicht der Zubereitung.

Entsprechend der erfindungsgemäßen Verwendung können die kosmetischen Desodorantien in Form von Aerosolen, also aus Aerosolbehältern, Quetschflaschen oder durch eine Pumpvorrichtung versprühbaren Präparaten vorliegen oder in Form von mittels Roll-On-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z.B. Cremes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Bevorzugt sind kosmetische Zubereitungen enthaltend Glycopyrrolat zu einem Anteil von 0,05 - 5,00 Gew.%, besonders bevorzugt 0,1 - 3,00 Gew.%, bezogen auf die Gesamtmasse der Zubereitung, in Kombination mit zwei der Aktivstoffe.

Besonders sind hierbei bevorzugt Zubereitungen in Form einer W/Si - Emulsion, eines O/W - Gel oder eines Seifengelstiftes.

Wie sich insbesondere anhand der Beispiele und anhand der exemplarisch dargestellten Wirksamkeitsstudien (Abbildung 1, 2 und 3) ablesen läßt, führt die Verwendung der Wirkstoffkombinationen, GLYCOPYRROLAT und ein oder mehrere Aktivstoffe, zur synergistischen Steigerung der antitranspirierenden und/oder desodorierende Wirkung im Vergleich zu Zubereitungen umfassend nur einen der Kombinationspartner.

Insbesondere ist die Deodorant und/oder Antitranspirantwirkung und die gleichzeitige hautpflegende Eigenschaft der erfindungsgemäßen Zubereitungen zu erwähnen.

In den nachfolgenden Beispielen, die bevorzugte Zubereitungen darstellen, sind die angegeben Anteile Gewichtsanteile bezogen auf die Gesamtmasse der Zubereitungen, sofern nichts anderes angegeben ist.

Beispielrezepturen

Beispiel 27

| Aerosolspray Typ A | Gew.-% |
|---|---|
| 2-Octyldodecanol | 0,50 |
| 1,2-Propylenglykol | 1,00 |
| 2-Butyloctansäure | 0,25 |
| Glycopyrroniumbromid | 0,50 |
| Parfum | q.s. |
| Ethanol | ad 100,00 |

Die durch Zusammenmischung der jeweiligen Bestandteile erhaltene flüssige Phase wird mit einem Propan-Butan-Gemisch (2.7) im Verhältnis 39:61 in Aerosolbehälter abgefüllt.

Beispiel 28

| Aerosolspray Typ B | I | II |
|---|---|---|
| | Gew.-% | Gew.-% |
| Aluminiumchlorohydrat | 45,00 | 25,00 |
| Isopropylpalmitat | 25,00 | 30,00 |
| Cyclomethicone | ad 100,00 | 0,30 |
| Isoparaffin | - | ad 100,00 |
| Talkum | - | 10,00 |
| 2-Hexyldecansäure | 0,25 | 0,30 |
| Glycopyrroniumbromid | 0,50 | 0,20 |
| Parfum | q.s. | q.s. |

Die durch Zusammenmischung der jeweiligen Bestandteile erhaltene flüssige Phase wird mit einem Propan-Butan-Gemisch (2.7) im Verhältnis 17:83 in Aerosolbehälter abgefüllt.

Beispiel 36

| Pumpzerstäuber | Gew.-% |
|---|---|
| Ethanol | 55,00 |
| PEG-40 Hydriertes Rizinusöl | 2,00 |
| Glycerin | 1,00 |
| 2-Butyloctansäure | 0,20 |
| Glycopyrroniumbromid | 0,60 |
| Parfum | q.s. |

(fortgesetzt)

| Pumpzerstäuber | Gew.-% |
|---|---|
| Wasser | ad 100,00 |

Beispiel 37

| Roll-on Gel | I | II |
|---|---|---|
| | Gew.-% | Gew.-% |
| Ethanol | 50,00 | 50,00 |
| PEG-40 Hydriertes Rizinusöl | 2,00 | 2,00 |
| Hydroxyethylcellulose | 0,50 | 0,50 |
| 2-Butyloctansäure | 0,20 | 0,30 |
| Glycopyrroniumbromid | 0,10 | 0,20 |
| Aluminiumchlorohydrat | - | 10,00 |
| Parfum | q.s. | q.s. |
| Wasser | ad 100,00 | ad 100,00 |

Beispiel 38

| Roll-on Emulsion: | I | II |
|---|---|---|
| | Gew.-% | Gew.-% |
| Aluminiumchlorohydrat | - | 10,00 |
| Polypropylenglykol(15)stearylether | 5,00 | 5,00 |
| Polyethylenglykol(100)stearylether | 1,00 | 1,00 |
| Polyethylenglykol(2)stearylether | 4,00 | 4,00 |
| 2-Hexyldecansäure | 0,20 | 0,30 |
| Glycopyrroniumbromid | 2,00 | 3,00 |
| Parfum, Konservierungsstoffe | q.s. | q.s. |
| Wasser | ad 100,00 | ad 100,00 |

Beispiel 39

| Deo-Stift Typ A | I | II | III |
|---|---|---|---|
| | Gew.-% | Gew.-% | Gew.-% |
| Natriumstearat | 7,00 | 7,00 | 7,00 |
| 1,2-Propylenglycol | 48,00 | 48,00 | 48,00 |
| Glycopyrroniumbromid | 0,20 | 0,30 | 0,30 |
| 2-Butyloctansäure | - | 0,10 | |

(fortgesetzt)

| Deo-Stift Typ A | I | II | III |
|---|---|---|---|
| | Gew.-% | Gew.-% | Gew.-% |
| 2-Hexyldecansäure | 0,20 | - | - |
| Parfum, Konservierungsstoffe | q.s. | q.s. | q.s. |
| Wasser | ad 100,00 | ad 100,00 | ad 100,00 |

Beispiel 40

| Deo-Stift Typ B | I | II | III |
|---|---|---|---|
| | Gew.-% | Gew.-% | Gew.-% |
| Natriumstearat | 8,00 | 8,00 | 8,00 |
| 1,2-Propylenglycol | 45,00 | 45,00 | 45,00 |
| Glycopyrroniumbromid | 0,20 | 0,30 | 0,30 |
| 2-Butyloctansäure | - | 0,50 | - |
| 2-Hexyldecansäure | 0,50 | - | - |
| Polyethylenglycol(25) cetearylether | 3,00 | 3,00 | 3,00 |
| Ethanol | 20,00 | 20,00 | 20,00 |
| Parfum, Konservierungsstoffe | q.s. | q.s. | q.s. |
| Wasser | ad 100,00 | ad 100,00 | ad 100,00 |

Alle dargestellten Beispielzusammensetzungen enthaltend die erfindungsgemäße Wirkstoffkombination aus Glycopyrrolat und mindestens einem der Aktivstoffe, ausgewählt aus der Gruppe einer oder mehrerer dialkylsubstituierter Essigsäuren, als kosmetische oder dermatologische Zubereitungen, diese enthaltend, die

- sich durch bessere Pflegewirkung auszeichnen,
- durch Hautneutralität das biologische Gleichgewicht der Haut weniger belasten,
- besser als Vehikel für kosmetische und medizinisch-dermatologische Wirkstoffe dienen,
- sich durch eine bessere physikochemische Stabilität der Formel auszeichnen
- sich durch bessere Bioverträglichkeit auszeichnen,
- eine verminderte Klebrigkeit auf der Haut aufweisen
- bessere sensorische Eigenschaften, wie beispielsweise die Verteilbarkeit oder das Einzugsvermögen in die Haut, besitzen,

als die Wirkstoffe, Wirkstoffkombinationen und Zubereitungen des Standes der Technik.

## Patentansprüche

1. Wirkstoffkombinationen aus Glycopyrroniumbromid in Kombination mit einem oder mehreren Aktivstoffen gewählt aus der Gruppe einer oder mehrerer dialkylsubstituierter Essigsäuren.

2. Wirkstoffkombinationen nach Anspruch 1, **dadurch gekennzeichnet, dass** die nachfolgenden Gewichtsverhältnisse eingestellt sind:

   (A : B : C) wie a : b : c gewählt wird, wobei a, b und c unabhängig voneinander positive rationale Zahlen von 1

bis 200, bevorzugt von 1 bis 50 darstellen,

A stellt dabei die der Konzentration von Glycopyrroniumbromid in Gewichtseinheiten (z.B. Gew.-%) dar, B stellt dabei die Konzentration des Aktivstoffes in denselben Gewichtseinheiten dar, C stellt dabei die Konzentration eines zweiten Aktivstoffes dar in denselben Gewichtseinheiten, gewählt aus der Gruppe

a.) Polyethylenglykol(2)stearylether und Polyethylenglykol (21)stearylether,
b.) grenzflächenaktiver Substanzen A, gewählt aus der Gruppe der Glucosederivate, welche sich durch die Strukturformel

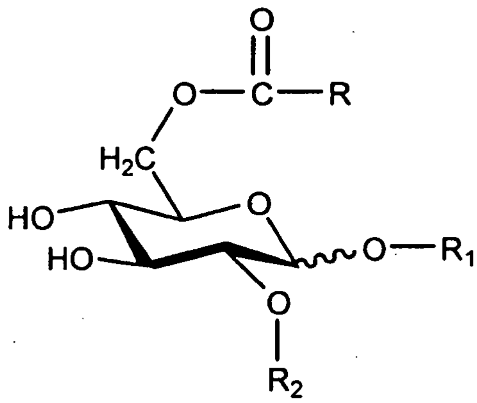

auszeichnen, wobei R einen verzweigten oder unverzweigten Alkylrest mit 1 bis 24 Kohlenstoffatomen darstellt, wobei R1 entweder ein Wasserstoffatom oder einen verzweigten oder unverzweigten Alkylrest mit 1 bis 24 Kohlenstoffatomen darstellt und wobei R2 entweder ein Wasserstoffatom oder einen verzweigten oder unverzweigten Acylrest mit 1 bis 24 Kohlenstoffatomen darstellt,
wobei zusätzlich eine oder mehrere grenzflächenaktive Substanz B enthalten sein können, gewählt aus der Gruppe der Substanzen der allgemeinen Strukturformel

$$R_3-O-(CH_2-CH(O-R_4)-CH_2-O)_n-R_5$$

,

wobei R3, R4 und R5 voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt,
c.) eines oder mehrerer partiell neutralisierten Ester von Monoglyceriden und/oder Diglyceriden gesättigter Fettsäuren mit Zitronensäure,
d.) eines oder mehrerer Polyole aus der Gruppe umfassend Ethylenglykol, Glycerin, Octoxyglycerin (2-Ethylhexylglycerinether) und $C_4$ -$C_{12}$ - Alkandiole,
e.) eines oder mehrerer Hydrokolloide, gewählt aus der Gruppe der organisch natürlichen oder modifiziert natürlichen, organisch vollsynthetischen oder anorganisch wasserlöslichen Polymere,
f.) eines oder mehrerer Mono-, Oligo- und/oder Polysaccharide,
g.) der Silikate in Kombination mit Ölen,
h.) der Öle, die gewählt werden aus aus der Gruppe der Alkylbenzoate, Esteröle, Dialkylcarbonate und -ether, lineare und/oder verzweigtkettige, aliphatische Kohlenwasserstoffe und/oder kurzkettige Kohlenwasserstoffester, insbesondere Isopropylester,
i.) der Parfumstoffe gewählt aus der Gruppe der Aledhyde, Ester, Jonone, Methyljonone, Damascone, Salicylate, Acetale und/oder Holzkörper,
j.) des Phenoxyethanol und/oder

k.) antimikrobiell wirksame silberhaltige Gläser.

**3.** Wirkstoffkombinationen nach Anspruch 2, bei denen der Quotient ( B + C) / A aus dem Bereich zwischen 0,5 und 200, bevorzugt aus dem Bereich zwischen 1 und 50 gewählt wird.

**4.** Wirkstoffkombination nach einem der Ansprüche 1 bis 3, **dadurch gekennzeichnet, dass** dialkyl-substituierte Essigsäuren der Formel

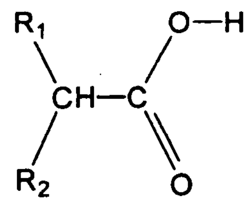

,

wobei $R_1$ einen verzweigten oder unverzweigten Alkylrest mit 1 - 12 Kohlenstoffatomen und $R_2$ einen verzweigten oder unverzweigten Alkylrest mit 1 - 24 Kohlenstoffatomen darstellt, gewählt werden.

**5.** Wirkstoffkombination nach einem der Ansprüche 1 bis 4, **dadurch gekennzeichnet, dass** als dialkyl-substituierte Essigsäuren 2-Butyloctansäure, 2-Butyldecansäure, 2-Hexyloctansäure und/oder 2-Hexyldecansäure, bevorzugt 2-Butyloctansäure, gewählt werden.

**6.** Kosmetische Zubereitungen umfassend Wirkstoffkombinationen gemäß einem der vorstehenden Ansprüche

**7.** Zubereitung nach Anspruch 6 umfassend keine weiteren Antitranspirantmittel.

**8.** Zubereitung nach Anspruch 6 umfassend 0 Gew.% Aluminiumhaltigen Antitranspirantien, insbesondere Aluminiumchlorhydrate.

**9.** Zubereitung nach einem der Ansprüche 6 bis 8, **dadurch gekennzeichnet, dass** die Zubereitung transparent gestaltet ist, insbesondere transparente W/Si oder O/W-Gele.

**10.** Verwendung der Zubereitungen nach Anspruch 6 bis 9 als desodorierende Zubereitung.

**11.** Verwendung der Zubereitungen nach Ansprüch 10 in Form von Flüssig- oder Stückseifen, Aerosolen, Deo-Stifte, Crèmes, Lotionen, desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays.

**12.** Verwendung der Zubereitungen nach Anspruch 11 in Kombination mit Aerosolbehältern, Quetschflaschen, Pumpvorrichtungen oder Roll-On-Vorrichtungen.

**13.** Verwendung der Wirkstoffkombination nach einem der Ansprüche 1 bis 5 oder Zubereitungen nach einem der Ansprüche 6 bis 9 zur Steigerung der desodorierenden Wirkung im Vergleich zu Zubereitungen umfassend nur einen der Kombinationspartner.

**14.** Verwendung der Wirkstoffkombination nach einem der Ansprüche 1 bis 5 oder Zubereitungen nach einem der Ansprüche 6 bis 9 als Deodorant und/oder Antitranspirant mit gleichzeitig hautpflegenden Eigenschaften.

**Claims**

**1.** Active agent combinations of glycopyrronium bromide in combination with one or more active substances chosen from the group of one or more dialkyl-substituted acetic acids.

**2.** Active agent combinations according to Claim 1, **characterized in that** the following weight ratios are set:

(A : B : C) is chosen as a : b : c, in which a, b and c represent, independently of one another, positive rational numbers from 1 to 200, preferably from 1 to 50,

A in this connection represents the concentration of glycopyrronium bromide in weight units (e.g. % by weight),
B in this connection represents the concentration of the active substance in the same weight units,
C in this connection represents the concentration of a second active substance in the same weight units, chosen from the group
a.) of polyethylene glycol (2) stearyl ether and polyethylene glycol (21) stearyl ether,
b.) of surface-active substances A, chosen from the group of the glucose derivatives, which are **characterized by** the structural formula

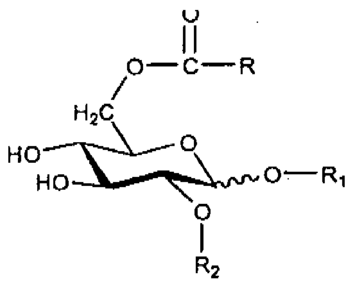

in which R represents a branched or unbranched alkyl radical with from 1 to 24 carbon atoms, in which $R_1$ represents either a hydrogen atom or a branched or unbranched alkyl radical with from 1 to 24 carbon atoms and in which $R_2$ represents either a hydrogen atom or a branched or unbranched acyl radical with from 1 to 24 carbon atoms,
in which, in addition, one or more surface-active substances B may be present, chosen from the group of the substances of the general structural formula

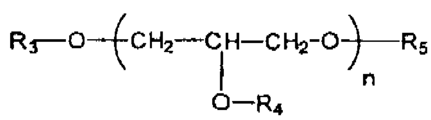

in which $R_3$, $R_4$ and $R_5$ are chosen, independently of one another, from the group which comprises: H and branched or unbranched and saturated or unsaturated fatty acid radicals with from 8 to 24 carbon atoms, in which up to three aliphatic hydrogen atoms can be replaced by hydroxyl groups, and n represents a number from 2 to 8,

c.) of one or more partially neutralized esters of monoglycerides and/or diglycerides of saturated fatty acids with citric acid,
d.) of one or more polyols from the group consisting of ethylene glycol, glycerol, octoxyglycerin (2-ethylhexyl glyceryl ether) and $C_4$-$C_{12}$-alkanediols,
e.) of one or more hydrocolloids, chosen from the group of the organically natural or modified natural, organically completely synthetic or inorganically water-soluble polymers,
f.) of one or more mono-, oligo- and/or polysaccharides,
g.) of the silicates in combination with oils,
h.) of the oils, which are chosen from the group of the alkyl benzoates, ester oils, dialkyl carbonates and ethers, linear and/or branched-chain aliphatic hydrocarbons and/or short-chain hydrocarbon esters, in particular isopropyl esters,
i.) of the perfume substances chosen from the group of the aldehydes, esters, ionones, methylionones, damascones, salicylates, acetals and/or wood bodies,
j.) of the phenoxyethanol and/or
k.) of antimicrobially effective silver- comprising glasses.

**3.** Active agent combinations according to Claim 2, in which the ratio (B + C)/A is chosen from the region between 0.5

and 200, preferably from the region between 1 and 50.

4. Active agent combination according to one of Claims to 3, **characterized in that** the choice is made of dialkyl-substituted acetic acids of the formula

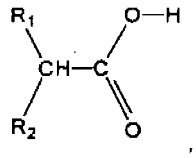

in which $R_1$ represents a branched or unbranched alkyl radical with from 1 to 12 carbon atoms and $R_2$ represents a branched or unbranched alkyl radical with from 1 to 24 carbon atoms.

5. Active agent combination according to one of Claims 1 to 4, **characterized in that** the choice is made, as dialkyl-substituted acetic acids, of 2-butyloctanoic acid, 2-butyldecanoic acid, 2-hexyloctanoic acid and/or 2-hexyldecanoic acid, preferably 2-butyloctanoic acid.

6. Cosmetic preparations comprising active agent combinations according to one of the preceding claims.

7. Preparation according to Claim 6, comprising no additional antiperspirants.

8. Preparation according to Claim 6, comprising 0% by weight of aluminium-comprising antiperspirants, in particular aluminium chlorohydrates.

9. Preparation according to one of Claims 6 to 8, **characterized in that** the preparation is fashioned transparently, in particular transparent W/Si or O/W gels.

10. Use of the preparations according to Claims 6 to 9 as deodorant preparation.

11. Use of the preparations according to Claim 10 in the form of liquid soaps, hard soaps, aerosols, deodorant sticks, creams, lotions, deodorant tinctures, deodorant intimate cleansing compositions, deodorant shampoos, deodorant shower or bath preparations, deodorant powders or deodorant powder sprays.

12. Use of the preparations according to Claim 11 in combination with aerosol containers, squeeze bottles, pump devices or roll-on devices.

13. Use of the active agent combination according to one of Claims 1 to 5 or preparations according to one of Claims 6 to 9 for increasing the deodorant action in comparison with preparations comprising only one of the combination partners.

14. Use of the active agent combination according to one of Claims 1 to 5 or preparations according to one of Claims 6 to 9 as deodorant and/or antiperspirant with simultaneously skin-care properties.

**Revendications**

1. Combinaisons de substances actives constituées par du bromure de glycopyrronium en combinaison avec une ou plusieurs substances actives choisies dans le groupe d'un ou de plusieurs acides acétiques substitués par dialkyle.

2. Combinaisons de substances actives selon la revendication 1, **caractérisées en ce que** les rapports pondéraux suivants sont réglés :

(A:B:C) est choisi comme a:b:c, où a, b et c représentent, indépendamment l'un de l'autre, des nombres rationnels positifs de 1 à 200, de préférence de 1 à 50,

A représente la concentration en bromure de glycopyrronium en unités pondérales (par exemple en % en

poids),
B représente la concentration en substance active dans les mêmes unités pondérales,
C représente la concentration en une deuxième substance active dans les mêmes unités pondérales, choisie dans le groupe
a.) des polyéthylèneglycol(2)stéaryléthers et des polyéthylèneglycol (21) stéaryléthers,
b.) des substances tensioactives A, choisies dans le groupe des dérivés de glucose, qui se distinguent par la formule développée

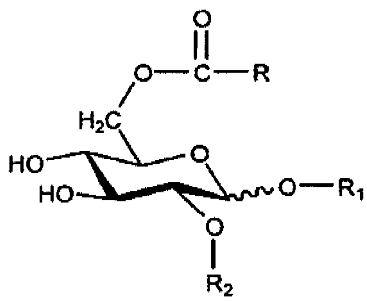

- où R représente un radical alkyle ramifié ou non ramifié comprenant 1 à 24 atomes de carbone, où $R_1$ représente soit un atome d'hydrogène, soit un radical alkyle ramifié ou non ramifié comprenant 1 à 24 atomes de carbone et où $R_2$ représente soit un atome d'hydrogéne, soit un radical acyle ramifié ou non ramifié comprenant 1 à 24 atomes de carbone,
- où en outre une ou plusieurs substances tensioactives B peuvent être contenues, choisies dans le groupe des substances de formule de structure générale

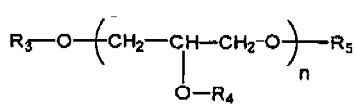

- où $R_3$, $R_4$ et $R_5$ sont choisis, indépendamment l'un de l'autre, dans le groupe comprenant : H, les radicaux d'acides gras ramifiés ou selon le cas non ramifiés, saturés ou selon le cas insaturés, comprenant 8 à 24 atomes de carbone, dans lesquels jusqu'à trois atomes d'hydrogène aliphatiques peuvent être substitués par des groupes hydroxy et n vaut un nombre de 2 à 8,

c.) d'un ou de plusieurs esters partiellement neutralisés de monoglycérides et/ou de diglycérides d'acides gras saturés avec de l'acide citrique,
d.) d'un ou de plusieurs polyols du groupe comprenant l'éthylèneglycol, le glycérol, l'octoxyglycérol (2-éthylhexylglycéroléther) et les $C_4$-$C_{12}$- alcanediols,
e.) d'un ou de plusieurs hydrocolloïdes, choisis dans le groupe des polymères organiquement naturels ou naturels modifiés, organiquement totalement synthétiques ou inorganiquement solubles dans l'eau,
f.) d'un ou de plusieurs monosaccharides, oligosaccharides et/ou polysaccharides,
g.) des silicates en combinaison avec des huiles,
h.) des huiles qui sont choisies dans le groupe des benzoates d'alkyle, des huiles d'ester, des dialkylcarbonates et des dialkyléthers, des hydrocarbures aliphatiques linéaires et/ou à chaîne ramifiée et/ou des esters d'hydrocarbures à courte chaîne, en particulier les esters isopropyliques,
i.) des substances parfumées choisies dans le groupe des aldéhydes, des esters, des ionones, des méthylionones, des damascones, des salicylates, des acétals et/ou des corps creux,
j.) du phénoxyéthanol et/ou
k.) des verres contenant de l'argent à action antimicrobienne.

**3.** Combinaisons de substances actives selon la revendication 2, dans lesquelles le quotient (B + C)/A est choisi dans la plage entre 0,5 et 200, de préférence dans la plage entre 1 et 50.

**4.** Combinaisons de substances actives selon l'une quelconque des revendications 1 à 3, **caractérisées en ce qu'**on choisit des acides acétiques substitués par dialkyle de formule

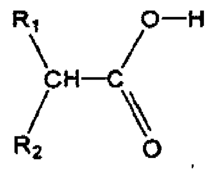

où $R_1$ représente un radical alkyle ramifié ou non ramifié comprenant 1 à 12 atomes de carbone et $R_2$ représente un radical alkyle ramifié ou non ramifié comprenant 1 à 24 atomes de carbone.

**5.** Combinaisons de substances actives selon l'une quelconque des revendications 1 ou 4, **caractérisées en ce qu'**on choisit comme acides acétiques substitués par dialkyle l'acide 2-butyloctanoique, l'acide 2-butyldécanoïque, l'acide 2-hexyloctanoïque et/ou l'acide 2-hexyldécanoïque, de préférence l'acide 2-butyloctanoïque.

**6.** Préparations cosmétiques comprenant des combinaisons de substances actives selon l'une quelconque des revendications précédentes.

**7.** Préparation selon la revendication 6 ne comprenant pas d'autres agents antiperspirants.

**8.** Préparation selon la revendication 6 comprenant 0% en poids d'antiperspirants contenant de l'aluminium, en particulier les chlorhydrates d'aluminium.

**9.** Préparation selon l'une quelconque des revendications 6 à 8, **caractérisée en ce que** la préparation est transparente, en particulier des gels transparents E/Si ou H/E.

**10.** Utilisation des préparations selon la revendication 6 à 9 comme préparation désodorisante.

**11.** Utilisation des préparations selon la revendication 10 sous forme de savons liquides ou en morceaux, d'aérosols, de bâtons déodorants, de crèmes, de lotions, de teintures désodorisantes, d'agents d'hygiène intime désodorisants, de shampooings désodorisants, de préparations pour douche ou bain désodorisantes, de poudres désodorisantes ou de sprays en poudre désodorisants.

**12.** Utilisation des préparations selon la revendication 11 en combinaison avec des récipients pour aérosol, des flacons souples, des dispositifs à pompe ou des dispositifs à bille.

**13.** Utilisation de la combinaison de substances actives selon l'une quelconque des revendications 1 à 5 ou des préparations selon l'une quelconque des revendications 6 à 9 pour augmenter l'action désodorisante par rapport aux préparations ne comprenant qu'un des partenaires de combinaison.

**14.** Utilisation de la combinaison de substances actives selon l'une quelconque des revendications 1 à 5 ou des préparations selon l'une quelconque des revendications 6 à 9 comme déodorant et/ou comme antiperspirant, présentant simultanément des propriétés de soin de la peau.

Abbildung 1

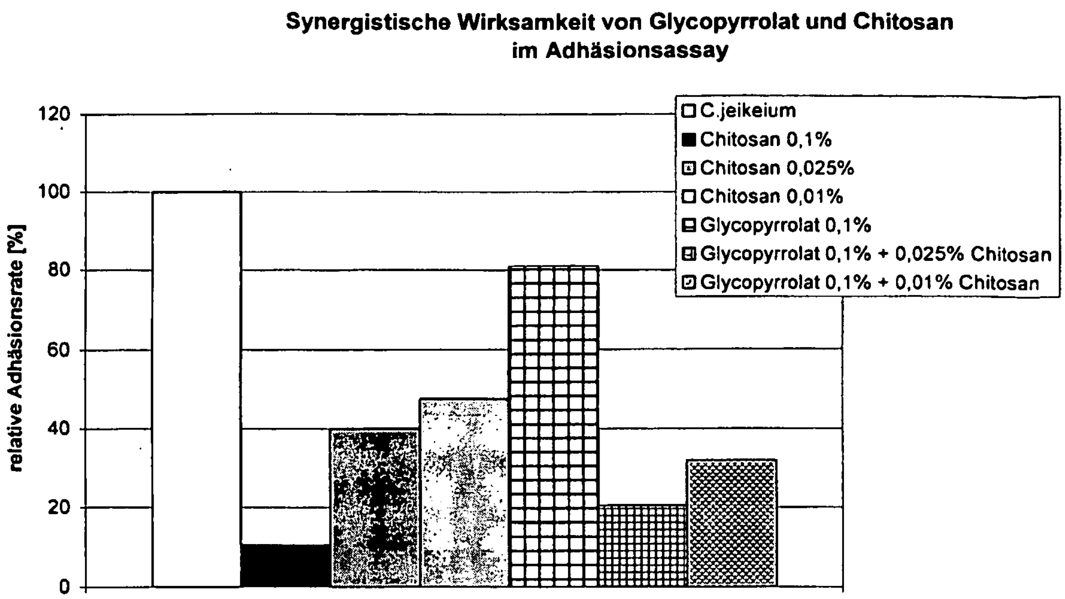
Synergistische Wirksamkeit von Glycopyrrolat und Chitosan
im Adhäsionsassay
relative Adhäsionsrate [%]
120
100
80
60
40
20
0
C.jeikeium
Chitosan 0,1%
Chitosan 0,025%
Chitosan 0,01%
Glycopyrrolat 0,1%
Glycopyrrolat 0,1% + 0,025% Chitosan
Glycopyrrolat 0,1% + 0,01% Chitosan

Abbildung 2

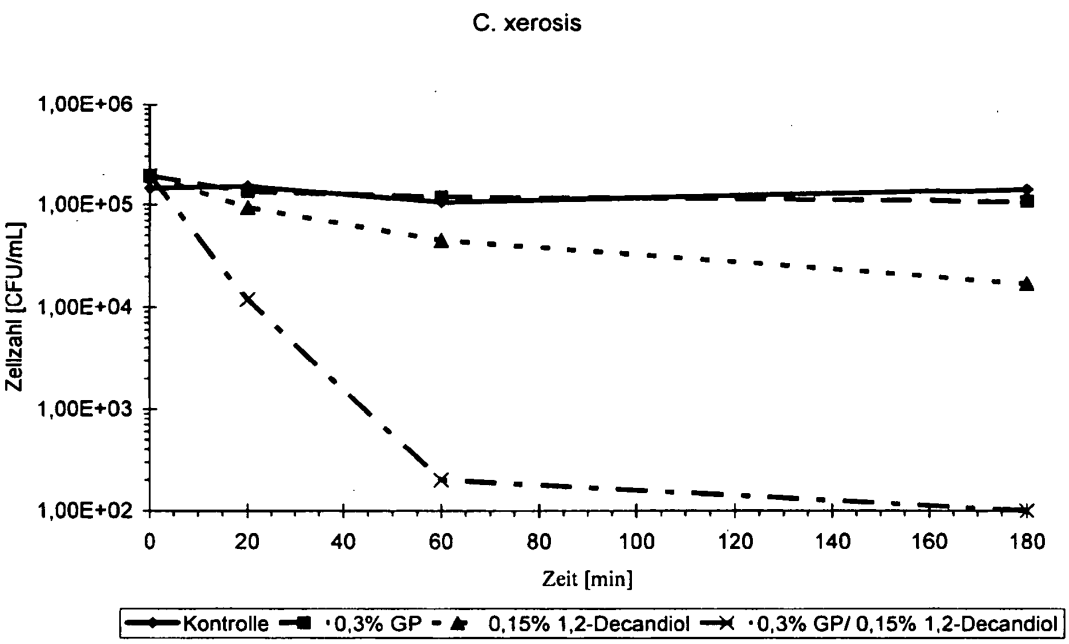
C. xerosis
1,00E+06
1,00E+05
1,00E+04
1,00E+03
1,00E+02
Zellzahl [CFU/mL]
0
20
40
60
80
100
120
140
160
180
Zeit [min]
Kontrolle
0,3% GP
0,15% 1,2-Decandiol
0,3% GP/ 0,15% 1,2-Decandiol

Abbildung 3

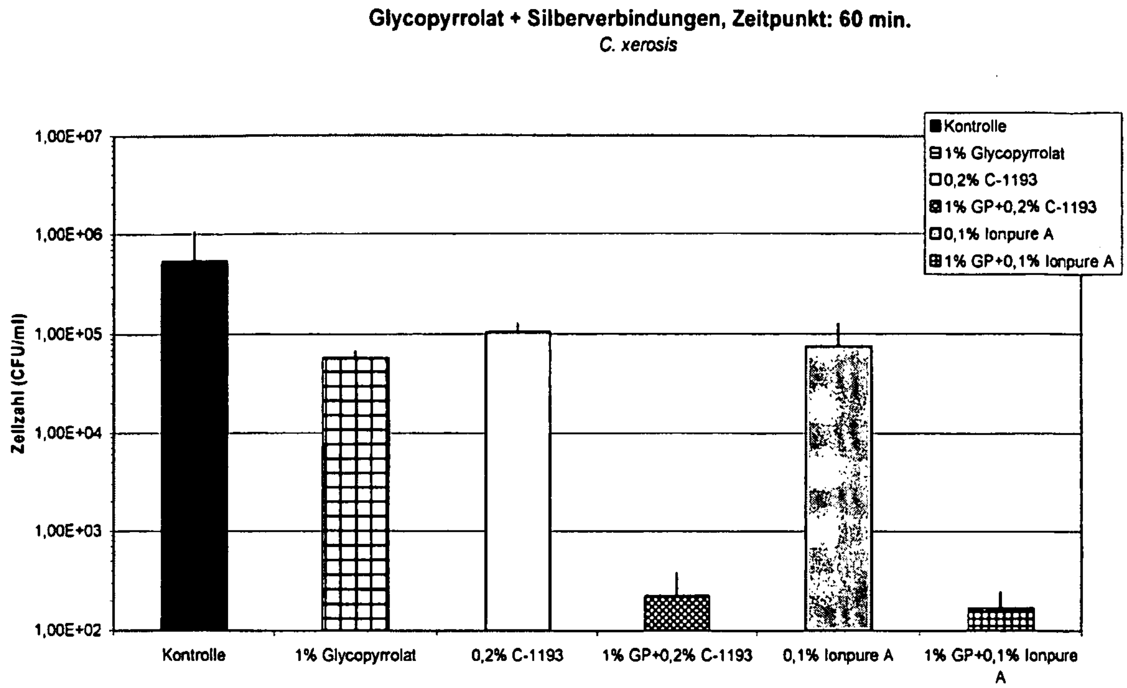
Glycopyrrolat + Silberverbindungen, Zeitpunkt: 60 min.
C. xerosis
Kontrolle
1% Glycopyrrolat
0,2% C-1193
1% GP+0,2% C-1193
0,1% Ionpure A
1% GP+0,1% Ionpure A
1,00E+07
1,00E+06
1,00E+05
1,00E+04
1,00E+03
1,00E+02
Zellzahl (CFU/ml)
Kontrolle
1% Glycopyrrolat
0,2% C-1193
1% GP+0,2% C-1193
0,1% Ionpure A
1% GP+0,1% Ionpure A

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03026585 A
- GB 1080960 A1
- WO 0108681 A
- US 6433033 B1
- WO 03011340 A
- DE 19516705 A1
- DE OS19516705 A
- EP 776657 A
- DE 4225045 A
- DE 4241118 A
- EP 619111 A
- DE 4224761 A
- DE 4314305 A
- DE 3627970 A
- DE 2917504 A
- EP 0319168 A
- DE 4009347
- DE 3740186
- DE 3938140
- DE 4204321
- DE 4229707
- DE 4229737
- DE 4237081
- DE 4309372
- DE 4324219


### In der Beschreibung aufgeführte Nicht-Patentliteratur


- *Anaesthesia,* 1983, vol. 38, 1195-1204
- **L.V.Allen.** Topical Agent Stops Facial Sweating. *Pharmacist,* Juli 1998
- **C.L. Hays et al.** The Frey Syndrome: A Simple, Effective Treatment. *Otolaryngol Head Neck Surg.,* 1982, vol. 90, 419-425
- **H.P.Fiedler.** Lexikon der Hilfsstoffe für Pharmazie, Kosmetik und angrenzende Gebiete. Editio Cantor, 1989, 293